(19) **Europäisches Patentamt**

**European Patent Office**

**Office européen des brevets**

(11) **EP 1 152 796 B1**

(12) **EUROPÄISCHE PATENTSCHRIFT**

(45) Veröffentlichungstag und Bekanntmachung des Hinweises auf die Patenterteilung:
**18.08.2004   Patentblatt 2004/34**

(51) Int Cl.⁷: **A61N 5/10**

(21) Anmeldenummer: **00907494.9**

(86) Internationale Anmeldenummer:
**PCT/EP2000/000857**

(22) Anmeldetag: **03.02.2000**

(87) Internationale Veröffentlichungsnummer:
**WO 2000/048676 (24.08.2000 Gazette 2000/34)**

(54) **VERFAHREN ZUR ÜBERPRÜFUNG EINER NOTABSCHALTUNG EINES IONENSTRAHL-THERAPIESYSTEMS**

METHOD FOR MONITORING AN EMERGENCY SWITCH-OFF OF AN ION-BEAM THERAPY SYSTEM

PROCEDE DE SURVEILLANCE DE L'ARRET D'URGENCE D'UN SYSTEME DE THERAPIE PAR FAISCEAU IONIQUE

(84) Benannte Vertragsstaaten:
**AT BE CH CY DE DK ES FI FR GB GR IE IT LI LU MC NL PT SE**

(30) Priorität: **19.02.1999   DE 19907064**

(43) Veröffentlichungstag der Anmeldung:
**14.11.2001   Patentblatt 2001/46**

(73) Patentinhaber: **Gesellschaft für Schwerionenforschung mbH 64291 Darmstadt (DE)**

(72) Erfinder:
• **BADURA, Eugen**
  **D-64291 Darmstadt (DE)**
• **BECHER, Wolfgang**
  **D-64291 Darmstadt (DE)**
• **BRAND, Holger**
  **D-64291 Darmstadt (DE)**
• **ESSEL, Hans-Georg**
  **D-64291 Darmstadt (DE)**
• **HABERER, Thomas**
  **D-64291 Darmstadt (DE)**
• **OTT, Wolfgang**
  **D-64291 Darmstadt (DE)**
• **POPPENSIEKER, Klaus**
  **D-64291 Darmstadt (DE)**

(74) Vertreter: **Boeters, Hans Dietrich, Dr. et al Patentanwälte Boeters & Bauer, Bereiteranger 15 81541 München (DE)**

(56) Entgegenhaltungen:
**US-A- 5 585 642**

• **NATH ET AL: "AAPM code of practice for radiotherapy accelerators" MEDICAL PHYSICS, - Juli 1994 (1994-07) Seiten 1093-1121, XP000460220**
• **PEDRONI E ET AL: "THE 200-MEV PROTON THERAPY PROJECT AT THE PAUL SCHERRER INSTITUTE: CONCEPTUAL DESIGN AND PRACTICAL REALIZATION" MEDICAL PHYSICS,US,AMERICAN INSTITUTE OF PHYSICS. NEW YORK, Bd. 22, Nr. 1, 1. Januar 1995 (1995-01-01), Seiten 37-53, XP000505145 ISSN: 0094-2405**
• **WEINHOUS ET AL: "Testing of a medical linear accelerator's computer-control system" MEDICAL PHYSICS, Bd. 17, Nr. 1, - 1990 Seiten 95-102, XP000101939**
• **MATSUURA J: "Systems for overall control and beam transport of the HIMAC" MITSUBISHI ELECTRIC ADVANCE, - September 1995 (1995-09) XP000905408**

**Beschreibung**

Die vorliegende Erfindung betrifft ein Verfahren zur Überprüfung einer Notabschaltung eines Ionenstrahl-Therapiesystems, welches insbesondere mit Schwerionen betrieben wird.

Nath et al., AAPM code of practice for radiotherapy accelerators, Medical Physics, Juli 1994, Seiten 1093-1121 offenbart ein Verfahren zur Überprüfung einer Notabschaltung eines Strahl-Therapiesystems, wobei eine Überprüfung eine Notabschaltung durchgeführt wird, unter automatischer Überwachung sicherheitsrelevanter Geräte parameter und unter Anzeigen sicherheitsrelevanter Zustände an Konsolen.

Ionenstrahl-Therapiesysteme werden bevorzugt zur Behandlung von Tumoren eingesetzt. Sie besitzen den Vorteil, daß bei Bestrahlung eines Zielobjekts (Targets) der größte Teil der Energie des Ionenstrahls auf das Target übertragen wird, während lediglich eine geringe Energie auf gesundes Gewebe übertragen wird. Demzufolge kann eine relativ hohe Bestrahlungsdosis zur Behandlung eines Patienten eingesetzt werden. Röntgenstrahlen übertragen hingegen ihre Energie gleichermaßen auf das Target sowie gesundes Gewebe, so daß aus gesundheitlichen Gründen zum Schutz des Patienten keine hohe Bestrahlungsdosis verwendet werden kann.

Aus der US-Patentschrift 4,870,287 ist beispielsweise ein Ionenstrahl-Therapiesystem bekannt, bei dem von einer Protonenquelle Protonenstrahlen erzeugt werden, deren Protonen über eine Beschleunigereinrichtung verschiedenen Behandlungs- bzw. Bestrahlungsplätzen zugeführt werden können. An jedem Behandlungsplatz ist ein Drehgerüst mit einer Patientenliege vorgesehen, so daß der Patient mit dem Protonenstrahl unter unterschiedlichen Bestrahlungswinkeln bestrahlt werden kann. Während sich der Patient innerhalb des Drehgerüsts räumlich an einer festen Stelle befindet, rotiert das Drehgerüst um den Körper des Patienten, um die Behandlungsstrahlen unter verschiedenen Bestrahlungswinkeln auf das im Isozentrum des Drehgerüsts befindliche Target zu fokussieren. Die Beschleunigereinrichtung umfaßt die Kombination aus einem linearen Beschleuniger (Linear Accelerator, LINAC) und einem sog. Synchrotronring.

In H.F. Weehuizen et al, CLOSED LOOP CONTROL OF A CYCLOTRON BEAM FOR PROTON THERAPY, KEK Proceedings 97-17, Januar 1998 wird ein Verfahren zur Stabilisierung des Protonenstrahls in Protonenstrahl-Therapiesystemen vorgeschlagen, wobei der Behandlungsstrahl aktiv derart gesteuert wird, daß er an zwei voneinander in Längsrichtung beabstandeten Meßpunkten auf der Mittellinie des entsprechenden Strahlzufuhrsystems liegt. Der erste Meßpunkt liegt zwischen einem Paar von Ablenkmagneten und ist durch eine Vieldraht-Ionisationskammer gebildet. Abhängig von dem von dieser Vieldraht-Ionisationskammer gelieferten Istwert der Strahlposition bezüglich des Mittelpunkts des Strahlpfads wird eine PI-Regelung von weiteren Ablenkmagneten, die vor dem erstgenannten Paar von Ablenkmagneten angeordnet sind, erzeugt. Der zweite Meßpunkt liegt kurz vor dem Isozentrum und ist durch eine in vier Quadranten unterteilte Ionisationskammer gebildet. Abhängig von dem Positionsistwert dieser Ionisationskammer werden wiederum PI-Regelsignale erzeugt, die jedoch für die erstgenannten Ablenkmagnete bestimmt sind. Mit Hilfe dieser Regelung soll sowohl eine Winkelstabilität bezüglich der Mittellinie des Strahlzufuhrsystems als auch eine laterale Positionsstabilität des Protonenstrahls möglich sein.

Bei Durchführung einer Schwerionenbestrahlung, d.h. einer Bestrahlung mit Ionen, die schwerer als Protonen sind, sind jedoch große und schwere Einrichtungen erforderlich, so daß hier die Tendenz besteht, den Einsatz von Drehgerüsten zu vermeiden und statt dessen den Patienten bzw. die Patientenliege zu bewegen. Entsprechende Therapiesysteme sind beispielsweise in E. Pedroni: Beam Delivery, Proc. 1st Int. Symposium on Hadrontherapy, Como, Italy, October 18-21, 1993, Seite 434 beschrieben. Bei diesen Systemen handelt es sich demnach um exzentrische Systeme.

Da jedoch von Onkologen grundsätzlich isozentrische Systeme bevorzugt werden, wurde ein Schwerionenstrahl-Therapiesystem vorgeschlagen, bei dem zwar Drehgerüste an den Behandlungsplätzen eingesetzt werden, jedoch die Radien der Drehgerüste dadurch verringert werden können, daß der jedem Drehgerüst horizontal entlang seiner Drehachse zugeführte Behandlungsstrahl mit Hilfe von geeigneten Magnet- und Optikanordnungen derart geführt wird, daß er zunächst von der Drehachse wegläuft und später wieder die Drehachse im Isozentrum zur Bestrahlung eines Targets kreuzt. Zur Bestrahlung des Targets ist ein Rasterscanner vorgesehen, der vertikale Ablenkmittel sowie horizontale Ablenkmittel umfaßt, die jeweils die Behandlungsstrahlen senkrecht zur Strahlachse ablenken, so daß eine das Target umgebende Fläche mit den Behandlungsstrahlen abgetastet wird. Dieses System sieht somit im wesentlichen eine Strahlführung in lediglich einer Ebene des Drehgerüsts vor.

Die Bestrahlung durch den Rasterscanner erfolgt mit Hilfe von Bestrahlungsdosisdaten, welche von dem Kontrollsystem des Ionenstrahl-Therapiesystems automatisch abhängig von dem zu bestrahlenden bzw. zu behandelnden Patienten berechnet werden.

Da bei Ionenstrahl-Therapiesystemen grundsätzlich eine hohe Betriebssicherheit und Betriebsstabilität hinsichtlich des Behandlungsstrahls erforderlich ist, ist bei dem zuvor beschriebenen Schwerionenstrahl-Therapiesystem eine Überwachungseinrichtung zum Überwachen des von dem Rasterscanner gelieferten Behandlungsstrahls vorgesehen. Diese Überwachungseinrichtung ist zwischen dem letzten Ablenkmagneten der oben genannten Magnetanordnung und dem Isozentrum angeordnet und kann Ionisationskammern zur Überwachung des Teilchenflusses und Vieldrahtkammern zur Überwachung der Strahlposition und der Strahlbreite umfassen.

Beim Betrieb von medizinischen Elektronenbeschleunigern sind aus Sicherheitsgründen verschiedene DIN-Normen einzuhalten. Diese betreffen zum einen die Abnahmeprüfung, d.h. die Überprüfung der Betriebsbereitschaft, und zum anderen die Konstanzprüfung, d.h. die Überprüfung der Betriebsstabilität, des Systems. Für Ionenstrahl-Therapiesysteme, insbesondere für Schwerionenstrahl-Therapiesysteme, sind derartige eigens für Ionenstrahl-Therapiesysteme entwickelte Sicherheitsnormen noch nicht bekannt. Auch bei Ionenstrahl-Therapiesystemen besteht jedoch das Bedürfnis nach einer größtmöglichen Betriebssicherheit und Betriebsstabilität.

Der vorliegenden Erfindung liegt daher die Aufgabe zugrunde, ein Verfahren zur Überprüfung einer Notabschaltung eines Ionenstrahl-Therapiesystems vorzuschlagen, um die Betriebssicherheit und Betriebsstabilität, insbesondere bezogen auf die Notabschaltung, zu verbessern. Dabei soll das Verfahren insbesondere zur Verwendung mit Schwerionen geeignet sein.

Diese Aufgabe wird gemäß der vorliegenden Erfindung durch ein Verfahren mit den Merkmalen des Anspruches 1 gelöst. Die abhängigen Ansprüche definieren jeweils bevorzugte und vorteilhafte Ausführungsformen der vorliegenden Erfindung.

Gemäß der vorliegenden Erfindung wird ein Ionenstrahl-Therapiesystem betrieben, welches mindestens eine Ionenquelle, eine Beschleunigungseinrichtung und ein Strahlführungssystem aufweist, wobei eine Überprüfung einer Notabschaltung durchgeführt wird.

Dazu wird eine Überprüfung einer manuellen und einer automatischen Notabschaltung unter automatischer Überwachung aller sicherheitsrelevanter Geräteparameter und unter Anzeige aller sicherheitsrelevanter Zustände an Konsolen eines technischen Kontrollraumes der Therapie und eines Hauptkontrollraumes des Gesamtsystems einer Ionenstrahl-Therapieanlage durchgeführt.

Diese Überprüfung der Funktionsfähigkeit einer Interlockeinheit oder einer Notabschaltung eines Ionenstrahl-Therapiesystems ist von hoher sicherheitstechnischer Relevanz.

So müssen beispielsweise alle sicherheitsrelevanten Geräteparameter auf die Auslösung einer Notabschaltung des Systems bei Vorliegen eines Interlockfalls oder einer Interlockbedingung überprüft werden. Eine Abschaltung des Behandlungsstrahls kann nur erfolgen, wenn ein Interlockfall erkannt wird. Daher müssen alle Quellen, die zu einem Interlockfall führen können einzeln in einem Test simuliert und die Auslösung des Interlocks, d.h. die Erzeugung der zur Notabschaltung des Behandlungsstrahls führenden Signale durch die Interlockeinheit, überprüft werden. Die Interlockeinheit überwacht im Betrieb vorzugsweise die Signale der Endschalter der beweglichen Teile in der Strahlführung, die Zustände der Magnetnetzgeräte der Rasterscannermagneten, die Ionisationskammern hinsichtlich der Spannungsversorgung, eines Datenüberlaufs der Datenübertragung, der Einhaltung der Intensitätsgrenzwerte und des Gleichlaufs der einzelnen Ionisationskammern, die Elektronik der Strahlpositionsmeßeinrichtung sowie die Strahlposition selbst, die Hochspannung und den Gasfluß der einzelnen Detektoren, einen möglichen Interlock durch den Ablaufsteuerungsrechner, die Position der Patientenliege, eine mögliche Unterbrechung der Immobilisierung des Patienten (z.B. bei Öffnen der Maske am Bestrahlungsplatz oder bei Bewegung des Patienten), die Funktionsbereitschaft aller Rechnerprogramme und eine mögliche Notabschaltung oder Freigabe einer Bestrahlung durch die Medizinbedienkonsole des Therapiesystems etc.. Bei fehlender Auslösung des Interlocks bei Vorliegen eines Interlockzustands muß in das Therapiesystem eingegriffen und der Fehler behoben werden. Diese Überprüfung sollte zur Konstanzprüfung täglich durchgeführt werden.

Ebenso muß die Funktionsfähigkeit der manuellen Notabschaltung über die Medizinbedienkonsole überprüft werden, da eine manuelle Notabschaltung jederzeit gewährleistet sein muß.

Schließlich ist auch eine Überprüfung der Anzeigen aller sicherheitsrelevanten Zustände an den einzelnen Konsolen des Ionenstrahl-Therapiesystems, insbesondere der technischen Kontrollräume und des Hauptkontrollraums vonnöten. Die Anzeige dieser sicherheitsrelevanten Zustände dient einer schnellen Fehlererkennung sowie Fehlerbehebung und geben dem Betriebspersonal Informationen über den momentanen Stand der Bestrahlung. Diese Anzeigen der Alarmbedingungen können zusammen mit dem oben beschriebenen Test der Interlockeinheit überprüft werden. Zur Konstanzprüfung sollte dieser Test vor jedem Bestrahlungsblock und nach jeder Änderung des Kontrollsystems oder der Programme durchgeführt werden.

Insbesondere wird vorgeschlagen, die berechneten Bestrahlungsdosiswerte für mehrere Meßpunkte des Phantoms zu überprüfen, wobei auf eine ausreichende Genauigkeit der Berechnung der Bestrahlungsdosisdaten geschlossen wird, falls die mittlere Abweichung zwischen den berechneten und gemessenen Werten der Bestrahlungsdosis für sämtliche Meßpunkte einen vorgegebenen ersten Toleranzwert und für jeden einzelnen Meßpunkt die Abweichung zwischen der für diesen Meßpunkt berechneten und gemessenen Bestrahlungsdosis einen vorgegebenen zweiten Toleranzwert nicht überschreitet. Dabei beträgt der erste Toleranzwert ±5 % und der zweite Toleranzwert ±7 %.

Zur Überprüfung einer korrekten Übertragung der geometrischen Strukturen am Behandlungsplatz sowie der Planungsparameter von einer bildgebenden Einrichtung des Ionenstrahl-Therapiesystems bis zur Positionierung kann von dem Phantom eine digitale Rekonstruktion, insbesondere eine Röntgen-Rekonstruktion, berechnet werden, die mit einer von dem Phantom erzeugten Röntgenaufnahme verglichen wird, um eine mögliche Abweichung festzustellen.

Die vorliegende Erfindung ermöglicht eine deutliche Verbesserung der Betriebsstabilität und Betriebssicherheit des Ionenstrahl-Therapiesystems und definiert einen Prüfplan mit bestimmten Prüfaspekten, welche im Sinne einer Abnahmeprüfung und/oder einer Konstanzprüfung des Ionenstrahl-Therapiesystems durchgeführt werden können. Dies betrifft insbesondere die Bestrahlungsplanung, in deren Verlauf automatisch Bestrahlungsdosisdaten in dem Ionenstrahl-Therapiesystem abhängig von dem zu bestrahlenden bzw. behandelnden Patienten berechnet werden.

Die Erfindung wird nachfolgend anhand eines bevorzugten Ausführungsbeispiels unter Bezugnahme auf die beigefügte Zeichnung beschrieben.

Fig. 1 zeigt eine vereinfachte schematische Darstellung einer bei dem vorliegenden Ionenstrahl-Therapiesystem eingesetzten Beschleunigungseinrichtung,

Fig. 2 zeigt eine Ansicht eines bei dem vorliegenden Ionenstrahl-Therapiesystem eingesetzten Drehgerüsts.

Ein der vorliegenden Erfindung zugrundeliegendes Ionenstrahl-Therapiesystem wird in der Regel in Krankenhausgebäuden eingesetzt, welche in einen medizinischen Bereich und einen Beschleunigerbereich unterteilt sind. Zur Behandlung von Patienten sind mehrere Behandlungs- oder Bestrahlungsplätze vorgesehen. Das Kontrollsystem des Ionenstrahl-Therapiesystems umfaßt mehrere Kontrollräume, wobei für die einzelnen Behandlungsplätze technische Kontrollräume und ein Hauptkontrollraum für die Beschleunigereinrichtung vorgesehen sein können. Darüber hinaus können in dem Gebäude Laboratorien für die Dosimetrie oder zur Beschleunigerwartung oder eine PET-Einrichtung (Positron-Emitter-Tomograph) untergebracht sein. Zudem sind Energieversorgungseinrichtungen (insbesondere für die Beschleunigereinrichtung und das Bestrahlungssystem) und Kühleinrichtungen vorgesehen. Die einzelnen Behandlungsräume sind durch dicke Wände und Decken begrenzt, die beispielsweise aus Beton mit einer Dicke von 2 m bestehen, um eine ausreichende Abschirmwirkung sicherzustellen.

Da der Grundaufbau des Ionenstrahl-Therapiesystems im wesentlichen nicht Thema der vorliegenden Erfindung ist, wird an dieser Stelle lediglich kurz darauf eingegangen.

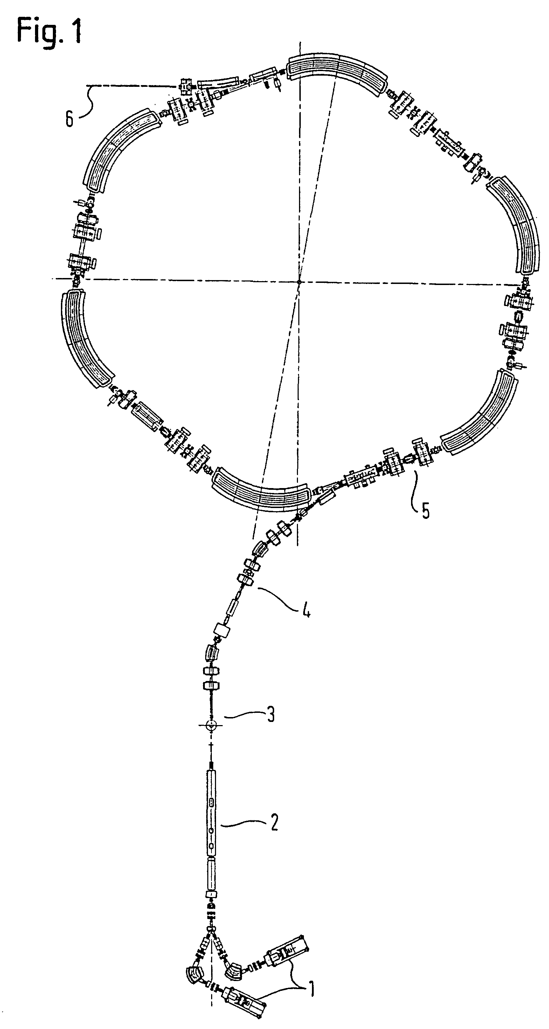

Das Ionenstrahl-Therapiesystem umfaßt ein Injektionssystem, welches zusammen mit der bereits zuvor erwähnten Beschleunigereinrichtung in Fig. 1 vereinfacht dargestellt ist.

Das Injektionssystem umfaßt Ionenquellen 1, deren Strahlung jeweils über Niederenergie-Strahlführungsleitungen mit einer Anordnung von Spektrometermagneten und Quadrupolen einem Schaltmagneten zugeführt werden, welcher die Strahlung u.a. über eine weitere Quadrupolanordnung und eine zur Pulsformung vorgesehenen Chopperanordnung einem linearen Beschleuniger 2 (Linear Accelerator, LINAC) zuführt.

Bei dem vorliegenden Ausführungsbeispiel sollen ausschließlich $^{12}C^{2+}$-Ionen verwendet werden, die in der Strahlführung zwischen dem linearen Beschleuniger 3 und dem Synchrotronring 5 auf $^{12}C^{6+}$ gestrippt werden. Zu diesem Zweck ist nach dem linearen Beschleuniger 2 ein Stripper 3 vorgesehen. Diese Kohlenstoffionen haben sich aufgrund ihrer physikalischen und biologischen Eigenschaften als sehr effektiv bei der Behandlung von Tumoren herausgestellt und besitzen die Vorteile einer hohen physikalischen Selektivität sowie einer hohen biologischen Effektivität und bieten darüber hinaus die Möglichkeit, die Bestrahlung mit Hilfe eines Positron-Emitter-Tomographen (PET) zu verifizieren. Durch die geeignete Auswahl der Kohlenstoffionen kann die biologische Effektivität derart gesteuert werden, daß sie im Plateaubereich der Bragg'schen Kurve gering und im Bereich des Bragg-Peaks hoch ist. Dadurch kann das Target bzw. der Tumor mit einer höheren Dosis behandelt werden, während die Dosis für das umgebende gesunde Gewebe minimiert wird.

Um die Verwendung bzw. Beschleunigung ausschließlich der beabsichtigten Ionensorte sicherzustellen, wird in dem Hochladungs-Injektionssystem ein Ladungsspektrum des vorliegenden Strahls aufgenommen und ausgewertet. Durch Vergleich des aufgenommenen Ladungsspektrums mit einem Referenzspektrum können ungewünschte Ionen oder Störstellen erkannt und entsprechende Maßnahmen ergriffen werden. Diese Überprüfung kann beispielsweise mit jedem Hochfahren einer Ionenquelle 1 durchgeführt werden.

Der lineare Beschleuniger 2 dient zur ersten Beschleunigung der ihm zugeführten Ionen, welche anschließend über eine Injektionsleitung 4 dem Synchrotron 5 zugeführt werden. Die Injektionsleitung 4 umfaßt neben dem bereits erwähnten Stripper 3 eine weitere Chopperanordnung zur Feinformung der Injektionspulse, Dipolmagnete zur Ladungsanalyse, Quadrupole zur Anpassung der Strahlung an das Aufnahmevermögen des Synchrotrons 5 etc..

Das Injektionssystem, welches u.a. die Ionenquellen 1, die Niederenergie-Strahlführungsleitungen, den linearen Beschleuniger 2 (LINAC), den Stripper 3 und die Injektionsleitung 4 umfaßt, besitzt somit insgesamt die Aufgabe, Ionenstrahlen mit gewünschten Teilchen zu erzeugen und zu analysieren, die Verunreinigung der Ionenstrahlen zu überwachen und die Ionenstrahlintensität zu steuern, die Ionen auf eine bestimmte Injektionsenergie zu beschleunigen sowie die Pulslänge der in den Synchrotronring 5 injizierten Pulse zu bestimmen.

Der Synchrotronring 5 dient zu abschließenden Beschleunigung der ihm zugeführten Ionen auf eine bestimmte Energie und umfaßt beispielsweise mehrere Ablenkmagnete, Quadrupole und Sextupole. Bei dem in Fig. 1 gezeigten Ausführungsbeispiel sind beispielsweise sechs Ablenkmagnete mit einem Ablenkwinkel von jeweils 60° vorgesehen. Innerhalb des Synchrotrons 5 ist eine (nicht gezeigte) Kühleinrichtung angeordnet. Durch mehrfache Injektionsumläufe werden die injizierten Ionen von einer Energie im Bereich von einigen MeV/u auf eine Energie von beispielsweise über 400 MeV/u beschleunigt. Der auf diese Weise beschleunigte Behandlungsstrahl wird an einer bestimmten Stelle des Synchrotrons über eine Hochenergie-Strahlführungsleitung 6 extrahiert und den einzelnen Behandlungsplätzen zugeführt.

Obwohl im allgemeinen die horizontale und vertikale Strahlausbreitung am Behandlungsplatz unterschiedlich ist, kann den Forderungen nach einer "idealen" symmetrischen und stabilen Strahlform am Behandlungsplatz im wesentlichen durch eine geeignete Einstellung der Strahloptik in den Strahlführungsleitungen Rechnung getragen werden.

Die Hochenergie-Strahlführungsleitung 6 umfaßt Quadrupollinsen, Ablenkmagnete, Strahlanalyseeinrichtungen usw.. Darüber hinaus kann ein weiterer Chopper hinter der Extraktionsstelle des Synchrotrons 5 angeordnet sein, der in Notfällen zum Einsatz kommt, um die Strahlzufuhr zu unterbrechen. Daneben kann eine routinemäßige Unterbrechung des Extraktionsvorgangs, der zum Auskoppeln des Behandlungsstrahls aus dem Synchrotron 5 dient, nach jedem Rasterscanabschnitt vorgesehen sein.

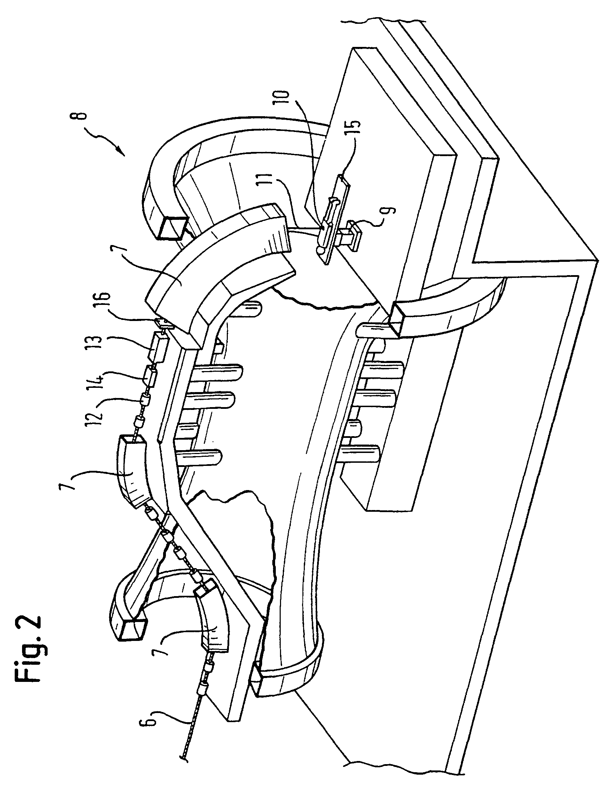

Fig. 2 zeigt eine perspektivische Ansicht eines der Drehgerüste 8, die jeweils an einem der Behandlungsplätze vorgesehen sind, denen der Behandlungsstrahl über die zuvor beschriebene Hochenergie-Strahlführungsleitung 6 zugeführt wird. Das Drehgerüst 8 rotiert um eine bestimmte Drehachse, während ein zu behandelnder Patient auf einer Patientenliege 9 mit örtlich fester Orientierung bzw. Ausrichtung liegt. Die zu behandelnde Körperstelle des Patienten befindet sich dabei im Isozentrum 10 des Behandlungsstrahls, wobei das Isozentrum als Schnittpunkt zwischen dem Zentralstrahl 11 des nachfolgend noch näher beschriebenen Rasterscanners und einer Drehachse der Patientenliege 9 definiert ist.

Wie aus Fig. 2 ersichtlich ist, ist die Hochenergie-Strahlführungsleitung 6 derart ausgestaltet, daß der Behandlungsstrahl nach Eintritt in das Drehgerüst 8 in einer Ebene mehrmals abgelenkt wird. Zu diesem Zweck sind mehrere Quadrupollinsen 12 und Dipolmagnete 7 vorgesehen, wobei die ersten beiden Dipolmagnete 7 identische Ablenkwinkel, beispielsweise 42°, aufweisen und zueinander entgegengesetzt angeordnet sind, während es sich bei dem letzten Dipolmagnet 7 um einen Ablenkmagnet mit einem Ablenkwinkel von 90° handelt, so daß der Behandlungsstrahl 11 nach Eintritt in das Drehgerüst 8 zunächst aus der Drehachse des Drehgerüsts 8 seitlich abgelenkt und anschließend parallel zur Drehachse des Drehgerüsts 8 geführt wird, um anschließend aus dem letzten Ablenkmagneten 7 über eine Strahlaustrittsöffnung unter einem Winkel von 90° bezüglich der Patientenliege 9 auszutreten.

Die bei dem vorliegenden Ionenstrahl-Therapiesystem vorgesehene Rasterscanneranordnung ist bei dem in Fig. 2 gezeigten Ausführungsbeispiel zwischen der letzten Quadrupollinse 12 und dem letzten Ablenkmagneten 7 des Drehgerüsts 8 angeordnet und umfaßt mindestens einen horizontalen Rasterscannermagneten 13 sowie mindestens einen vertikalen Rasterscannermagneten 14. Die Rasterscannermagnete 13 und 14 lenken den Ionenstrahl 11 jeweils senkrecht zur Strahlachse 11 entweder horizontal oder vertikal ab, so daß der auf diese Weise gerasterte Ionenstrahl 11 nach Austritt aus dem letzten Ablenkmagneten 7 in Übereinstimmung mit einem vorgegebenen Behandlungsplan eine bestimmte, das Isozentrum 10 umgebende Fläche abtastet. Aufgrund der Anordnung des Rasterscanners 13, 14 zwischen dem letzten Quadrupolmagneten 12 und dem letzten Ablenkmagneten 7 kann eine hohe Flexibilität bei der nachfolgend noch näher beschriebenen Regelung der Strahlgröße und Strahldispersion am Isozentrum 10 erzielt werden.

Die Rasterscannermagnete 13, 14 werden von einer (nicht gezeigten) Steuereinrichtung angesteuert, welche Bestandteil des Gesamtkontrollsystems des Ionenstrahl-Therapiesystems ist.

Im Bereich zwischen der Strahlaustrittsöffnung des letzten Ablenkmagneten 7 und dem Isozentrum 10 sind Überwachungsmittel zur Überwachung des Behandlungsstrahls 11 vorgesehen. Diese Überwachungsmittel, welche beispielsweise zur Erfassung und Regelung der Strahlposition, Strahlform und des Teilchenflusses vorgesehen sind, werden nachfolgend noch ausführlich erläutert.

Wie bereits zuvor erwähnt worden ist, kann zusätzlich ein Positron-Emitter-Tomograph (PET) zur Überwachung des Bestrahlungsvorgangs vorgesehen sein, dessen Bildaufnehmer (Kamera) in einer In-Strahl-Position ausgerichtet ist. Die Positron-Emitter-Tomographie wird vorzugsweise während der Behandlung bzw. Bestrahlung durchgeführt. Trifft ein Behandlungsstrahl auf Gewebe, werden ausgehend von den Primärionen Positronen emittierende Isotope erzeugt. Einige dieser Isotope, welche sich von den Primärionen lediglich durch den Verlust von ein oder zwei Neutronen unterscheiden, stoppen nahezu in demselben Bereich wie die entsprechenden Primärionen. Dieser Stoppunkt der sogenannten Positronenemitter kann zur Überwachung des Bestrahlungsvorgangs mit Hilfe der Positron-Emitter-Tomographie ermittelt werden.

Für das zuvor beschriebene Ionenstrahl-Therapiesystem wurde ein umfangreiches Prüfsystem zur Überprüfung und Regelung der wesentlichen Leistungsmerkmale des Therapiesystems entwickelt, auf welches nachfolgend näher eingegangen werden soll.

Ein erster Abschnitt des Prüfsystems betrifft die Erzeugung des Behandlungsstrahls 11.

Neben der bereits zuvor beschriebenen Überprüfung der Ionensorte wird dabei auch die Strahlenergie des Behandlungsstrahls überwacht. Dies ist erforderlich, da die von der jeweiligen Therapie geforderten Strahlenergien eingehalten werden müssen. Zu diesem Zweck umfassen die in Fig. 2 angedeuteten Überwachungsmittel ein Absorber-Ionisationskammersystem, welches am Isozentrum 10 des jeweiligen Behandlungsplatzes anzuordnen ist. Das Absorber-Ionisationskammersystem mißt für einige ausgewählte Energiestufen, welche während eines Therapie-Testzyklus aktiviert werden, die Lage des Bragg-Peaks am Behandlungsplatz, wobei sich die augenblickliche Strahlenergie aus der gemessenen Position des Bragg-Peaks ergibt. Zur Bestimmung der Position des Bragg-Peaks werden die Bragg'schen-Kurven in feinen Schritten ausgemessen. Sollte sich bei der Überprüfung eine Abweichung des Bragg-Peaks von der Sollposition um mehr als 0,5mm ergeben, ist eine Intervention erforderlich. Zu Konstanzprüfung kann der zuvor beschriebene Prüfvorgang vor jedem Bestrahlungsblock durchgeführt werden.

Ein weiterer Unterpunkt hinsichtlich der Überprüfung des Behandlungsstrahls betrifft die Überwachung des Intensitätsniveaus des langsam extrahierten Behandlungsstrahls am Bestrahlungs- bzw. Behandlungsplatz. Die limitierte Dynamik des Rasterscanners begrenzt die Abtast- oder Scangeschwindigkeit des gerasterten Behandlungsstrahls nach oben, wobei die für diese Limitierung maßgebliche Komponente die maximale Stromanstiegsgeschwindigkeit der Magnetstromversorgungseinrichtungen ist. Die Scangeschwindigkeit des Behandlungsstrahls hängt von der jeweiligen Intensität des Strahls und der geplanten Teilchenbelegung ab. Um sicherzustellen, daß die maximale Scangeschwindigkeit während der Bestrahlung nicht erreicht wird, darf die aus dem Synchrotron 5 extrahierte Teilchenrate die Sollvorgabe nicht wesentlich überschreiten. Bei einer deutlichen Unterschreitung hingegen verlängert sich die Gesamtbestrahlungszeit, wobei gegebenenfalls dann das Kontroll- bzw. Überwachungs- oder Monitorsystem im Bereich sehr kleiner Eingangsströme betrieben wird, was eine Beeinträchtigung der Präzision des Strahlnachweises zur Folge haben kann. Daher ist beim vorliegenden Therapiesystem eine Messung und Protokollierung der Teilchenintensitäten im Synchrotron im oberen Intensitätsbereich sowie der dem Bestrahlungsplatz zugeführten Teilchenrate für alle Intensitätsschritte für mehrere Energie über einige Minuten vorgesehen. Die von dem Beschleuniger dem Bestrahlungsplatz zugeführte Teilchenrate liegt zwischen $2 \times 10^6$ und $2 \times 10^8$ Ionen pro Extraktion aus dem Synchrotron 5. Die Abweichung der Teilchenrate von der Sollvorgabe darf nach oben maximal 30% und nach unten maximal 50% betragen. Werden diese Grenzwerte überschritten, ist eine entsprechende Intervention erforderlich. Zur Konstanzprüfung des Therapiesystems kann diese Überprüfung beispielsweise täglich durchgeführt werden.

Bei der Datenversorgung der Beschleuniger, der Bestrahlungsplanung und der Rasterscan-Programmierung müssen die gleichen Abhängigkeiten der Energie-, Intensitäts- und Fokussierungsvariation zugrunde gelegt werden. Um dies sicherzustellen, sollten die nach der letzten Therapie-Programmierung beschleunigerseitig erstellten Dateieinträge mit denjenigen verglichen werden, die für die Rasterscan-Programmierung und Bestrahlungsplanung verwendet werden. Eine Abweichung dieser Dateieinträge ist nicht zulässig. Zur Konstanzprüfung sollte diese Überprüfung vor jedem Bestrahlungsblock durchgeführt werden.

Während einer Bestrahlung werden die für die Therapie erforderlichen Beschleunigerabschnitte gegen (externe) Eingriffe verriegelt, um bewußte und unbewußte Fehleinstellungen zu vermeiden. Gleichzeitig werden Betriebszustände aller Komponenten aktiviert und ausschließlich auf die in Speichern, beispielsweise EPROMS, abgelegten Gerätesolldaten zugegriffen. Die Funktion dieser Beschleuniger-Verriegelung gegen Eingriffe kann dadurch überprüft werden, daß ein "Superzyklus" erstellt wird, der sowohl Experiment- als auch Therapiebeschleuniger enthält. In die Hochenergie-Strahlführung 6 zum Drehgestell 8 werden Überwachungsmittel oder Detektoren, wie z.B. (nachfolgend noch näher beschriebene) Profilgitter, Leuchttargets und Ionisationskammern, eingefahren und strahlbeeinflussende Elemente der Hochenergie-Strahlführungsleitung 6 und des Synchrotrons 5 für den Therapiebeschleuniger deaktiviert. Anschließend wird die Beschleunigerverriegelung aktiviert und alle Experimentbeschleuniger deaktiviert, während der Therapiebeschleuniger aktiviert wird. Zudem werden für den Therapiebeschleuniger alle zuvor deaktivierten Komponenten aktiviert und die eingefahrenen Profilgitter, Leuchttargets und Ionisationskammern wieder hinausgefahren. Nunmehr werden an einzelne Magnete Ausschaltbefehle und an Strahlführungs-Diagnosekomponenten Stellbefehle abgesendet, die im Normalfall aufgrund der Verriegelung des Beschleunigers keine Auswirkungen haben dürfen. Ansonsten liegt ein Fehler vor, der entsprechend korrigiert werden muß. Diese Überprüfung kann zur Konstanzprüfung vor jedem Bestrahlungsblock durchgeführt werden.

Die Extraktion des Behandlungsstrahls aus dem Synchrotron 5 muß aus Sicherheitsgründen innerhalb weniger als 1ms nach einem entsprechenden Signal einer Interlockeinheit des Therapiesystems abgebrochen werden können. Dies geschieht durch ein schnelles Abschalten eines speziellen Quadrupols im Synchrotron 5. Die Zeit zwischen einer Anforderung zum Strahlabbruch durch das Kontroll- und Sicherheitssystem und dem Ausbleiben des Strahls am Bestrahlungsort ist von entscheidender Bedeutung sowohl für den Rasterscanvorgang beim Wechsel aufeinanderfolgender Isoenergieabschnitte, welche mit konstanter Energie zu bestrahlenden Flächen entsprechen, als auch für eine eventuelle Notabschaltung des Systems im Fehlerfall. Es ist daher ein Test vorgesehen, der die Gesamtzeit, d.h. sowohl die Reaktionszeit der Anforderung als auch die des Strahlabbruchs, mißt. Zu diesem Zweck erzeugt das Kontrollsystem ein entsprechendes Signal, welches die Beendigung eines Isoenergieabschnitts simuliert, oder es wird eine Interlockbedingung, d.h. eine Bedingung für eine Notabschaltung, erzeugt. Anschließend wird von dem Kontrollsystem die Teilchenzahl nach einem Abbruch gemessen, wobei diese 1ms nach dem Abbruch nicht größer als $10^4$ Teilchen/s sein darf. Zusätzlich wird mit einem Speicheroszillographen und einem Pulser, welche in dem technischen Kontrollraum des Therapiesystems fest installiert sind, eine Messung durchgeführt, die das Ausgangssignal des Strom-Spannungs-Wandlers einer der Ionisationskammern auswertet, um somit die zuvor beschriebene Messung des Kontrollsystems zu überprüfen. Auch bei dieser zweiten Messung darf somit 1ms nach Abbruch kein Strahl nachgewiesen werden können. Folgende Zeitpunkte eines Abbruchs sollten nacheinander überprüft werden: zu Beginn der Extraktionszeit, in der Mitte der Extraktionszeit, am Ende der Extraktionszeit und außerhalb der Extraktionszeit. Die Überprüfung sollte als Konstanzprüfung täglich durchgeführt werden.

Nach Beendigung jeder Bestrahlung muß von der Beschleunigerseite ein Protokoll erstellt werden, welches die Einstellungen wesentlicher Beschleunigerkomponenten während der Bestrahlung sowie ausgewählte Strahldiagnose-Meßergebnisse dokumentiert. Um die Funktionsfähigkeit der Protokollierung sowie den Protokollinhalt zu testen, wird vorgeschlagen, einen Referenz-Therapiezyklus zu aktivieren und das Protokollprogramm aufzurufen. Anschließend können die durch das Protokollprogramm erstellten Protokolldaten mit den erwarteten Daten verglichen werden, wobei bei Unvollständigkeit des Protokolls oder bei Vorliegen eines protokollierten Gerätefehlers eine Intervention erfolgen muß. Zur Konstanzprüfung kann dieser Prüfvorgang vor jedem Bestrahlungsblock durchgeführt werden.

Ein weiterer Abschnitt des Prüfsystems betrifft die Überprüfung der Führung des Behandlungsstrahls (vor dem Bestrahlungsplatz).

Vom Beschleuniger aus muß sichergestellt werden, daß bei einer Abbruchanforderung ein Extraktionsabbruch erfolgt ist. Sollte der Behandlungsstrahl nicht durch die Abbruchanforderung abgebrochen werden, wird dies von dem Kontroll- und Sicherheitssystem durch eine Intensitätsmessung festgestellt und über einen redundanten, getrennt vorgesehenen Kanal erneut ein Abbruch des Strahls angefordert. Diese zweite Anforderung wirkt auf einen entsprechenden Ablenkdipol der Hochenergie-Strahlführungsleitung 6. Um auch die Funktionsfähigkeit dieses redundanten Extraktionsabbruchs zu überprüfen, wird die für den ersten Extraktionsabbruch vorgesehene Alarmleitung künstlich unterbrochen. In diesem Fall müßte der zuvor beschriebene zweite Extraktionsabbruch automatisch ausgelöst werden, was analog zu der oben beschriebenen Überprüfung des eigentlichen Extraktionsabbruchs getestet werden kann. Kommt es innerhalb von 10ms nicht zu einem Extraktionsabbruch, ist ein entsprechender Eingriff erforderlich. Zur Konstanzprüfung kann dieser Test vor jedem Bestrahlungsblock durchgeführt werden.

Mit einem weiteren Test kann die Funktion der Zu- und Ausschaltung der in der Hochenergie-Strahlführungsleitung 6 angeordneten Dipole getestet werden. Aus Gründen der Patientensicherheit ist die Ausschaltung der letzten beiden Ablenkmagnete in der Hochenergie-Strahlführungsleitung 6 vor der Bestrahlung (nach der Beschleunigerverriegelung) nur von dem technischen Kontrollraum aus über spezielle Kabelverbindungen zum Netzgerät dieser Magnete aktivierbar. Durch diese Ausschaltung wird die Strahlzufuhr an den Bestrahlungsplatz unterbunden. Die Zuschaltung dieser Magnete kann nur von dem technischen Kontrollraum aus über ein spezielles Signal und nicht (wie üblich) vom Hauptkontrollraum des Beschleunigers aus erfolgen. Die Funktion dieser Zu- und Abschaltung wird getestet, wobei dabei auch die entsprechenden Verbindungen/Anschlüsse überprüft werden. Zur Konstanzprüfung wird dieser Test vor jedem Bestrahlungsblock durchgeführt.

Ein weiterer Abschnitt des Prüfsystems betrifft die Überprüfung der Strahlführung am Bestrahlungsplatz.

Gemäß einem ersten Aspekt dieses Prüfabschnitts wird die Nullpunktslage des Behandlungsstrahls überwacht. Um eine genaue Strahlpositionierung am Isozentrum 10 nach Ablenkung des Strahls 11 durch die Rasterscannermagnete 13, 14 zu gewährleisten, muß die Achslage des Behandlungsstrahls 11 im letzten Teil der Strahlführung zum Bestrahlungsplatz für den gesamten Energie- und Fokussierungsbereich überprüft werden. Zu diesem Zweck werden Profilgitter 16 hinter den Rasterscannermagneten 13 und 14 sowie am Strahlaustrittsfenster in den Strahlpfad eingefahren und Testzyklen über den gesamten Energie- und Fokussierungsbereich erzeugt. Dabei werden die Profilgitter einzeln ausgewertet und die dabei erfaßten Strahlparameter protokolliert. Bei der Messung des am Strahlaustrittsfenster angeordneten Profilgitters muß das davor angeordnete Profilgitter 16 herausgefahren werden. Durch Auswertung der von den Profilgittern gelieferten Strahlparameter kann sowohl in horizontaler als auch in vertikaler Richtung die Strahlposition und der Strahlwinkel bestimmt werden. Aus den Strahlpositionen der Profilgitter wird die zu erwartende Position des Behandlungsstrahls am Isozentrum 10 ermittelt und anschließend das Protokoll überprüft. Wird dabei für das Isozentrum 10 ein Positionsfehler von ±25 % bezüglich der geforderten Strahl-Halbwertsbreiten festgestellt, muß ein entsprechender Eingriff erfolgen. Zu Konstanzprüfung kann dieser Test täglich durchgeführt werden.

Gemäß einem weiteren Aspekt dieses Prüfabschnitts wird der absolute Strahlort sowie die Ortsstabilität des Behandlungsstrahls am Bestrahlungsplatz überprüft. Die Einhaltung der absoluten Strahlposition ist die Voraussetzung für die Umsetzung der Behandlungs- bzw. Bestrahlungspläne. Daher muß der absolute Ort mit ortsempfindlichen Detektoren des Kontrollsystems gemessen werden. Die relative Ortsstabilität des Behandlungsstrahls im Isozentrum des Bestrahlungsplatzes bestimmt die Genauigkeit, mit der ein Bestrahlungsplan durchgeführt werden kann. Der Ort des Behandlungsstrahls wird online, d.h. kontinuierlich, während einer Bestrahlung gemessen und überprüft. Bei Abweichungen vom Sollort innerhalb einer vom Bestrahlungsplan vorgegebenen Toleranzgrenze wird die Bestrahlung abgebrochen bzw. eine entsprechende Intervention getätigt. Jeder ortsempfindliche Detektor wird getrennt überprüft.

Die Prüfung wird mit einem Profilgitter und ortsempfindlichen Detektoren, wie z.B. Vieldrahtkammern, durchgeführt.

Bei Verwendung von Profilgittern wird die absolute Strahllage im Isozentrum 10 mit Hilfe eines Leuchttargets oder Films am Ort des Isozentrums überprüft. Dabei wird die Lage des Profilgitters mit dem durch ein Laserkreuz auf dem Leuchttarget oder Film sichtbar gemachten Isozentrum justiert. Mit den Rasterscannermagneten 13, 14 wird der Behandlungsstrahl 11 statisch in das Isozentrum 10 abgelenkt und die durch die Profilgittermessung erhaltenen Ortskoordinaten mit den vorgegebenen Sollwerten verglichen. Dies kann beispielsweise in regelmäßigen Abständen, z.B. bei etwa jedem zehnten Energieschritt, durchgeführt werden.

Bei Verwendung von Vieldrahtkammern zur online-Kontrolle und Regelung der Strahllage werden zwei Vieldrahtkammern in einem Abstand von etwa 970 mm und 790 mm vor dem Isozentrum 10 positioniert und mit Hilfe eines Laserstrahls derart ausgerichtet, daß der durch das Isozentrum 10 verlaufende Zentralstrahl senkrecht durch das Zentrum der Vieldrahtkammern verläuft. Mit den Rasterscannermagneten 13, 14 wird der Strahl beispielsweise bei fünf verschiedenen Energien auf jeweils fünf unterschiedliche Positionen innerhalb des Bestrahlungsbereichs (nämlich oben und unten jeweils links und rechts sowie in der Mitte) statisch abgelenkt. Der Ort der Einstellung wird von dem Kontrollsystem gemessen und mit den Sollwerten verglichen.

Da sich die Vieldrahtkammern in unterschiedlicher Entfernung vor dem Isozentrum befinden, wird das Bestrahlungsfeld in den beiden Vieldrahtkammern mit unterschiedlichen Faktoren verkleinert abgebildet. Durch Anwendung der Regeln der Strahlgeometrie und des Strahlensatzes ergeben sich folgende Verkleinerungsfaktoren:

Vieldrahtkammer 970 mm vor dem Isozentrum:

x-Koordinate: Verkleinerungsfaktor 0,890
y-Koordinate: Verkleinerungsfaktor 0,876

Vieldrahtkammer 790 mm vor dem Isozentrum:

x-Koordinate: Verkleinerungsfaktor 0,910
y-Koordinate: Verkleinerungsfaktor 0,899

Vor der Überprüfung der absoluten Strahlposition mit den Vieldrahtkammern sollte eine Kalibrierung ihrer absoluten Positionen durchgeführt werden. Zu diesem Zweck wird nach Ausrichtung und Fixierung der Vieldrahtkammern ein mittels des zuvor erwähnten Laserkreuzes absolut positionierter Film an fünf Positionen bestrahlt. Der anhand des Films ermittelte Strahl-Nullpunkt wird mit dem aus den Vieldrahtkammern berechneten verglichen. Die Differenz oder Abweichung ergibt dann Korrekturoffsetwerte für die Ortsberechnung. Diese Korrekturoffsetwerte werden in den Positionssollwerten berücksichtigt, wobei die absolute Position aller fünf Punkte miteinander verglichen wird.

Mit den auf dieser Weise kalibrierten Vieldrahtkammern wird anschließend die absolute Strahlposition überprüft, wobei eine Regelung derart durchgeführt wird, daß die auf diese Weise ermittelte Positionsabweichung maximal 25 % der Halbwertsbreite des Strahlprofils entspricht. Diese Interventionsschwelle relativ zur Halbwertsbreite des Strahlprofils erwies sich als praktikabel, da alle geometrischen Parameter eines Bestrahlungsplans mit der Halbwertsbreite skalieren und insbesondere die für den Patientenbetrieb erforderliche Qualität der erzeugten Teilchenbelegungen erreicht wird. Bei Durchführung einer Konstanzprüfung sollten lediglich die zuvor beschriebenen Vieldrahtkammermessungen eingesetzt werden, da die Installation eines zusätzlichen Profilgitters im Isozentrum für den täglichen Betrieb sehr aufwendig wäre.

Ein weiterer Aspekt dieses Prüfabschnitts umfaßt die Überwachung und Regelung der absoluten Strahlprofilbreite und der zeitlichen Stabilität. Die von der Beschleunigereinrichtung nach Anforderung durch eine Pulszentralenansteuerung des Kontrollsystems gelieferte Strahlfokussierung muß eingehalten werden, da auf diesen Werten die Behandlungs- bzw. Bestrahlungspläne beruhen. Zu diesem Zweck wird die absolute Strahlprofilbreite im Isozentrum 10 mit Hilfe eines Profilgitters überprüft, wobei die Lage des Profilgitters mit dem durch ein Laserkreuz auf einem Leuchttarget oder Film sichtbar gemachten Isozentrum justiert wird. Mit den Rasterscannermagneten 13, 14 wird der Behandlungsstrahl statisch in das Isozentrum abgelenkt, wobei dies beispielsweise bei etwa jedem zehnten Energieschritt erfolgen kann. Die mit der Profilgittermessung erhaltenen Strahlbreiten werden mit vorgegebenen Sollwerten verglichen, wobei eine Regelung derart erfolgt, daß eine maximale Abweichung der Strahlbreite von ±50 % von der Sollvorgabe eingehalten wird. Dies gilt insbesondere für den Energiebereich oberhalb 200 MeV/u.

Die Konstanzprüfung des Ionenstrahl-Therapiesystems kann wiederum mit den bereits zuvor beschriebenen Vieldrahtkammern erfolgen, die sich in einem Abstand von 970 mm bzw. 790 mm vor dem Isozentrum 10 befinden. Vor der eigentlichen Prüfung erfolgt eine Kalibrierung der absoluten Breitenmessung der beiden Vieldrahtkammern. Dabei wird ein Film mit horizontalen und vertikalen Streifen bestrahlt, wobei jeder Strahl mit einer Extraktion aus dem Synchrotron bei einer festen Fokussierung erzeugt wird. Auf diese Weise können abhängig von den auswählbaren Fokussierungen beispielsweise sieben Strahlen erzeugt werden. Die anhand des bestrahlten Films ermittelten Strahlbreiten werden mit den von den Vieldrahtkammern (Ortskammern) gemessenen verglichen, um daraus Korrekturoffsetwerte zu erhalten, welche anschließend wieder in den Sollwerten berücksichtigt werden können. Anschließend wird mit Hilfe der somit kalibrierten Vieldrahtkammern in Verbindung mit dem Kontrollsystem die Halbwertsbreite des Strahlprofils sowie dessen zeitliche Konstanz oder Stabilität gemessen und überwacht, wobei dies insbesondere bei verschiedenen Energien und Intensitäten für die jeweils auswählbaren Fokussierungen durchgeführt wird.

Die zuvor beschriebene Anhebung der Interventionsschwelle von 20 % auf 50 % der Halbwertsbreite bei der Messung der absoluten Strahlprofilbreite im Vergleich zur Messung des absoluten Strahlorts ist kompatibel mit der Homogenitätsanforderung, da der Abstand der Strahlpositionen im Rahmen der Bestrahlungsplanung auf 33 % der Halbwertsbreite gesetzt wird.

Vor dem Isozentrum befinden sich üblicherweise einige Elemente zur Analyse und Modulation des Behandlungsstrahls, wie beispielsweise das Strahlaustrittsfenster, Detektoren oder ein Ripple-Filter. Durch diese Elemente wird eine Aufstreuung des Behandlungsstrahls hervorgerufen, welche mit abnehmender Strahlenergie stark zunimmt. Dadurch kann aus physikalischen Gründen eine Einhaltung der ursprünglich geforderten Strahlbreite im unteren Energiebereich (Energien < 200 MeV/u) nicht mehr oder nur schwer realisierbar sein. In diesem Fall wäre eine Überschreitung der oberen Toleranzwerte die Folge, so daß dieser Effekt bei der Bestrahlungsplanung berücksichtigt werden muß.

Ein weiterer Aspekt dieses Prüfabschnitts betrifft die Überwachung der Teilchenzahl im Behandlungsstrahl, d.h. die Überwachung der Variation der Teilchenzahl. Um den Meßbereich für Teilchenzahlmessungen nicht zu groß werden zu lassen, sollte die Intensität des vom Beschleuniger gelieferten Behandlungsstrahls lediglich innerhalb bestimmter Toleranzgrenzen schwanken. Im vorliegenden Fall wird vorgeschlagen, die Intensität des Behandlungsstrahls mit Hilfe der Ionisationskammern in Verbindung mit der Meßapparatur des Kontrollsystems zu messen und die Teilchenzahl über ein Zeitfenster von 300 µs zu mitteln. Die anschließend gemessenen Teilchenzahlen dürfen innerhalb des Zeitfensters maximal dem fünffachen Wert des zuvor erfaßten Mittelwerts entsprechen, um nicht eine Intervention auszulösen. Durch diese Maßnahmen kann ein sicherer Meßbereich gewählt werden, mit dem auch Teilchenzahlen noch korrekt gemessen werden können, die beispielsweise um einen Faktor 10 höher als der zuvor berechnete Mittelwert sind. Sollten roch höhere Teilchenzahlen auftreten, wird ein Alarm ausgelöst, und die bereits erwähnte Interlockeinheit löst die Strahlabschaltung aus. Es ist jedoch zu beachten, daß dieser Prüfaspekt lediglich die Voreinstellung der Detektoren betrifft und keinen direkten Einfluß auf die Energiedosis oder dergleichen hat. Auch bei einer Variation der Teilchenzahl, die deutlich oberhalb der zuvor definierten Interventionsschwelle liegt, kann die noch später beschriebene Homogenität der Teilchenbelegungen als entscheidendes Qualitätskriterium ausreichend sein.

Schließlich sollten hinsichtlich einer sicheren und stabilen Strahlführung am Bestrahlungsplatz auch die Sollpositionen sämtlicher beweglicher Teile zwischen den letzten Ablenkmagneten der Hochenergie-Strahlführungsleitung 6 und dem Drehgestell 8 regelmäßig überprüft werden, da jeder sich in der Strahlführung befindliche Gegenstand zu einer Beeinträchtigung der Strahlqualität am Bestrahlungsplatz führt. Es muß daher sichergestellt werden, daß sich keine beweglichen Teile der Strahlführung im Strahlweg befinden. Zu diesem Zweck sind an den entsprechenden beweglichen Teilen Endschalter angebracht, deren Zustände durch das Kontrollsystem automatisch und einzeln überprüft werden können. Dies sollte zur Konstanzprüfung vor jedem Bestrahlungsblock wiederholt werden.

Ein vierter Abschnitt des Prüfsystems betrifft die Überprüfung von Merkmalen, welche mit der Bestrahlungssteuereinheit des Ionenstrahl-Therapiesystems zusammenhängen.

Die in den zuvor beschriebenen Ionisationskammern des Überwachungs- oder Monitorsystems des Therapiesystems erzeugte elektrische Ladung, welche zur Bestimmung der Teilchenzahl dient, hängt vom Druck und der Temperatur des Ionisationskammergases ab, so daß diese beiden Größen während der Bestrahlung überwacht und protokolliert werden müssen. Der Druck und die Temperatur des Gases der Ionisationskammern werden mit Hilfe von elektrischen Sensoren gemessen, wobei die Meßwerte etwa einmal pro Minute vom Kontrollsystem erfaßt und mit eingetragenen Kalibrierfaktoren in absolute Einheiten (hPa und °C) umgerechnet und digital angezeigt werden. Der zeitliche Verlauf der Meßwerte kann in einem Trend-Diagramm grafisch dargestellt werden. Die Sensoren werden mit Hilfe von Referenzmeßgeräten kalibriert. Die Kalibrierung der in den Ionisationskammern angebrachten Sensoren sollte vor jedem Therapie-Bestrahlungsblock wiederholt werden. Zusätzlich werden der Luftdruck und die Raumtemperatur am Ort des Überwachungssystems mit absolut kalibrierten Geräten gemessen und vom Kontrollsystem erfaßt sowie bei jeder Bestrahlung protokolliert. Somit können zur (täglichen) Überprüfung der Ionisationskammern die Absolutwerte für Luftdruck und Raumtemperatur direkt an den Referenzmeßgeräten abgelesen, mit den vom Kontrollsystem angezeigten Werten verglichen und protokolliert werden. Als Referenzwerte dienen dabei die bei der täglichen Kalibrierung des Überwachungssystems registrierten Meßwerte. Bei einer Abweichung von 20 hPa bzw. 5 °C wird vom Kontrollsystem ein Alarm ausgelöst.

Auch das Laden von Programmen und Datensätzen in die Steuerungsrechner des Ionenstrahl-Therapiesystems muß überprüft werden. Dies ist erforderlich, um Daten, welche für eine Patientenbestrahlung erforderlich sind, korrekt in die Ablaufsteuerung des Systems laden zu können. Nur wenn alle Daten korrekt sind, darf eine Patientenbestrahlung aufgenommen werden. Zu diesem Zweck werden mit Hilfe von speziellen Programmen in den Serverrechnern des Kontrollsystems Programme und Daten in die einzelnen Prozessoren der Steuerungsrechner geschrieben, zurückgelesen und mit den in den eigenen Speichern gespeicherten Programmen und Daten verglichen, wobei diese Prüfprogramme vor jeder Bestrahlung automatisch ausgeführt werden. Nur wenn die zurückgeladenen Daten genau den in den Datenspeichern des Kontrollsystems gespeicherten Daten entsprechen, kann von einem sicheren Betrieb ausgegangen werden. Bei Abweichungen wird eine Alarmmeldung generiert und die zuvor beschriebene Interlockeinheit, welche zur Unterbindung einer Bestrahlung dient, kann nicht freigegeben werden.

Ein weiterer Prüfaspekt betrifft das Schalten der Ströme für die Ablenkmagnete 13, 14 des Rasterscanners. Dabei muß sichergestellt werden, daß die Stromwerte dieser Ablenkmagneten einen bestimmten, bei den Magnetnetzgeräten eingestellten Sollwert sowohl wertmäßig als auch zeitlich innerhalb bestimmter Toleranzgrenzen erreichen. Zu diesem Zweck wird die Zeit zwischen dem Setzen eines Magnetstromwerts in den Magnetnetzgeräten und dem Erreichen des entsprechenden stabilen Magnetstroms für verschiedene Stromwerte gemessen. Die dabei tolerierbare maximale Stromgenauigkeit bezüglich einer Abweichung vom eingestellten Magnetstromwert beträgt 0,3 A. Die maximal tolerierbare Einstellzeit bei einem Stromsprung von 2 A beträgt 175 µs in x-Richtung und 325 µs in y-Richtung. Werden diese Toleranzen nicht eingehalten, muß die Bestrahlung abgebrochen werden. Zur Konstanzprüfung kann dieser Test vor jedem Bestrahlungsblock durchgeführt werden.

Schließlich muß auch sichergestellt werden, daß die Nummer des bei Auftreten einer Abbruchbedingung aktiven Bestrahlungspunktes permanent, d.h. gegen Stromausfall gesichert, gespeichert wird. Dies ermöglicht eine von autorisierten Personen genehmigte Fortsetzung der Bestrahlung zu einem späteren Zeitpunkt. Die Funktionsfähigkeit dieser implementierten Sicherungsfunktion kann dadurch überprüft werden, daß ein bestimmter Bestrahlungs- oder Behandlungsplan in das Kontrollsystem geladen und ohne Bestrahlung ausgeführt, d.h. simuliert wird. Bei einem bestimmten Bestrahlungsort wird die Spannungsversorgung der Ablaufsteuerung ausgeschaltet und nach Wiederanfahren des Systems der letzte Bestrahlungsort ausgelesen und mit dem Bestrahlungsort bei Abschaltung der Spannungsversorgung verglichen. Bei Nichtübereinstimmung erfolgt ein entsprechender Eingriff. Diese Überprüfung wird zur Konstanzprüfung vor jedem Bestrahlungsblock durchgeführt.

Ein weiterer Abschnitt des Prüfsystems betrifft die Überprüfung der medizinischen Einrichtungen zur Patientenpositionierung des Ionenstrahl-Therapiesystems.

So sollte beispielsweise die Genauigkeit der stereotaktischen Koordinatenbestimmung eines Zielpunkts mit Hilfe eines CToder MR-Verfahrens überprüft werden, da die Genauigkeit der stereotaktischen Bildgebung ein wesentlicher Faktor für die Gesamtgenauigkeit der Bestrahlung ist. Zu diesem Zweck kann ein beliebiger Zielpunkt innerhalb eines Kugelphantoms durch einen speziellen Prüfkörper repräsentiert werden, dessen Mittelpunkt gut sichtbar mit Hilfe der bildgebenden Verfahren darstellbar ist. Das Kugelphantom wird in den stereotaktischen Rahmen eingespannt, so daß der Mittelpunkt zu einem unbekannten Zielpunkt wird. Anschließend werden die stereotaktischen Koordinaten mit Hilfe der angewandten Röntgen-, CT- oder MR-Verfahren zeitlich nacheinander ermittelt, wobei bei den tomographischen Verfahren der Schichtabstand 1 mm betragen sollte. Da die Genauigkeit des Röntgenverfahrens auf 1/10 mm genau ist, kann die Genauigkeit der Zielpunktbestimmung mit CT und MR durch Vergleich mit dem Röntgenverfahren bestimmt werden, d.h. es wird der radiale Abstand zwischen der mit der Röntgenaufnahme ermittelten Position des Zielpunkts und der mit dem CT- bzw. MR-Verfahren ermittelten Position überprüft. Der radiale Abstand darf nicht mehr als 1,5 mm betragen. Zur Konstanzprüfung genügt die jährliche Durchführung dieses Tests.

Als weiterer Prüfaspekt wird vorgeschlagen, die Genauigkeit der Lage des Isozentrums zwischen der Drehachse der Patientenliege 9 und dem Zentralstrahl 11 des Rasterscanners 13, 14 zu überprüfen, da das als Schnittpunkt zwischen der Drehachse der Patientenliege 9 und dem Zentralstrahl 11 des Rasterscanners 13, 14 definierte Isozentrum das Verbindungselement bei der Positionierung zwischen Planung und Bestrahlung ist. Eine Konstanzprüfung sollte vor jedem Bestrahlungsblock durchgeführt werden.

Zur Überprüfung des Isozentrums in bezug auf die Drehachse der Patientenliege 9 wird ein metallischer Probekörper (2-3 mm Durchmesser) mit Hilfe von Lasern in das nominale Isozentrum, d.h. in die nominale Drehachse der Patientenliege 9, gebracht. Durch ein Senkblei, welches genau auf den Mittelpunkt oberhalb des Probekörpers gerichtet wird, wird die Position des Probekörpers festgehalten. Bei Rotation um die Drehachse der Patientenliege 9 wird das Ausmaß der Bewegung des Probekörpers in bezug auf das Senkblei ermittelt. Diese Prozedur wird in mindestens drei unterschiedlichen Höhen der Patientenliege 9, bei einer maximalen Verstellbarkeit der Patientenliege 9 um 15 cm nach oben/unten beispielsweise in Höhe des Isozentrum 10 sowie mit mindestens 15 cm Abstand nach oben und unten, durchgeführt. Die maximale Abweichung darf in Strahlrichtung 1,0 mm und senkrecht zur Strahlrichtung lediglich 0,5 mm betragen. Veränderungen in Strahlrichtung sind weniger kritisch, da Dosisverteilungen im Patienten nicht davon beeinflußt werden.

Zur Überprüfung des Isozentrums in bezug auf den Zentralstrahl 11 wird die Lage des Isozentrums per Definition auf der Drehachse der Patientenliege 9 unterhalb der Geradeaus-Strahlebene festgelegt und durch ein optisches Vermessungssystem in bezug auf Wandmarkierungen festgestellt. Die Prüfung der Position des Prüfkörpers in bezug auf den Zentralstrahl 11 erfolgt mit Hilfe einer Filmmessung, wobei ein Verifikationsfilm in Strahlrichtung gesehen hinter dem Probekörper mit einem (ungerasterten) Zentralstrahl bestrahlt wird, dessen Halbwertsbreite größer als der Durchmesser des Probekörpers ist, so daß sich die Position des Probekörpers auf dem Verifikationsfilm in bezug auf den Zentralstrahl abbildet. In diesem Fall liegt die Interventionsschwelle bei einer Abweichung von maximal 25 % der Halbwertsbreite des Primärstrahls.

Zudem muß die Genauigkeit der Laserjustierung auf das Isozentrum 10 überprüft werden, da die Laser das Isozentrum 10 markieren. Dabei werden die Laser nach Positionierung des Probekörpers in dem Isozentrum 10 mit Hilfe optischer Vermessung auf den Mittelpunkt des Probekörpers ausgerichtet und die Abweichung der Laserlinien von der Horizontalen bzw. Vertikalen überprüft, wobei die maximale Abweichung jeweils 1mm betragen darf. Für die Konstanzprüfung wird die Abbildung der Laser auf den gegenüberliegenden Wänden bzw. auf dem Fußboden markiert und dient anschließend als Bezugswert.

Ein weiterer Prüfaspekt betrifft die Genauigkeit der Justierung der Röntgenröhren und des Zielkreuzes auf den gegenüberliegenden Aufnahmestationen, da das Röntgenverfahren ein zusätzliches Verfahren zur Markierung des Isozentrums 10 darstellt. Nach Positionierung des Probekörpers in dem Isozentrum 10 mit Hilfe der optischen Vermessung, d.h. mit Hilfe der Laser, werden Röntgenaufnahmen in den drei Raumrichtungen durchgeführt und der Abstand der Abbildung zwischen dem Probekörper und dem Zielkreuz auf dem Röntgenbild ermittelt. Der Probekörper sollte sich exakt auf dem Bild des Zielkreuzes abbilden, so daß der maximale Abstand zwischen der Abbildung des Probekörpers und dem Zielkreuz 1mm betragen darf.

Aufgrund der isozentrischen Bestrahlung der Patienten muß auch die Genauigkeit der Anzeige der Winkelskala der isozentrischen Rotation der Patientenliege 9 überprüft werden, wobei dies analog zu den Vorschriften der DIN 6847-5, Punkt 12.2.4 erfolgen kann. Die maximal tolerierbare Ungenauigkeit beträgt 1°.

Ebenso sollte die räumliche Stabilität der isozentrischen Rotation der Patientenliege 9 überprüft werden, da die Definition des Isozentrums 10 eine entsprechende Stabilität voraussetzt. Diese Überprüfung kann analog zu DIN 6847-5, Punkt 14.2 durchgeführt werden, wobei die Interventionsschwelle bei einer Ungenauigkeit von 1 mm liegt.

Schließlich wird auch vorgeschlagen, die Genauigkeit der Patientenlagerung und -positionierung zu überprüfen, da eine exakte Patientenpositionierung Voraussetzung für eine tumorkonforme Bestrahlung ist. Zur Abnahmeprüfung und Konstanzprüfung (vor jedem Bestrahlungsblock) des Therapiesystems können diesbezüglich die unbekannten stereotaktischen Koordinaten des Mittelpunkts eines Probekörpers, der innerhalb des stereotaktischen Grundrings fixiert wurde, als Zielpunkt ermittelt und der Mittelpunkt mit Hilfe des stereotaktischen Zielgeräts und durch transversale Bewegung der Patientenliege 9 in das Isozentrum 10 gebracht werden. In dieser Position werden in den drei Raumrichtungen Röntgenaufnahmen durchgeführt und der Abstand der Lage des Prüfkörpers vom Zielkreuz auf den drei Aufnahmen ermittelt. Der radiale Abstand zwischen dem Mittelpunkt des Prüfkörpers und dem Isozentrum darf maximal 1,5 mm betragen. Ansonsten ist eine entsprechende Korrektur der Patientenlagerung notwendig.

Ein weiterer Abschnitt des Prüfsystems betrifft die Bestrahlungsplanung, in deren Verlauf insbesondere die für eine bestimmte Bestrahlung beabsichtigten Bestrahlungsdosiswerte berechnet werden.

Zunächst muß sichergestellt werden, daß für die Planung von Bestrahlungen, d.h. für die Berechnung jeder Bestrahlungsdosis, stets dieselben Basisdatensätze verwendet werden. Dies kann dadurch erfolgen, daß der Name, das Datum und die Größe der die Basisdaten enthaltenden Dateien mit den korrekten Bezeichnungen einer zuvor angelegten Sicherungskopie verglichen werden. Dies geschieht automatisch bei jedem Aufruf des Dosisberechnungsalgorithmus.

Auch die Identität der Werte der aktuellen Basisdatensätze mit den entsprechenden Werten einer Sicherungskopie muß überprüft werden, um sicherzustellen, daß die Basisdatensätze nicht unkontrolliert verändert worden sind. Auch hier erfolgt der Vergleich des Inhalts der aktuellen Basisdatensätze mit der Sicherungskopie mit Hilfe eines Computerprogramms, welches insbesondere vor jedem Bestrahlungsblock gestartet werden sollte.

Nach DIN 6873 Teil 5, Bestrahlungsplanungssysteme, ist zudem eine Prüfung der Bezugswerte im Basisdatensatz einmal im Monat erforderlich. Dieser Unterpunkt kann bei der vorliegenden Bestrahlungsplanung mit Schwerionen entfallen, da die Tiefendosisverteilungen, d.h. die Energieverlustdaten als Funktion der Tiefe, als absolute Werte in bezug auf die Eingangsfluenz abgespeichert sind. Somit ist kein besonderer Bezugswert für die Dosis aufgezeichnet. Die verwendeten Basisdatensätze werden bereits wie oben beschrieben überprüft.

Ein wesentlicher Aspekt bei der Prüfung der Bestrahlungsplanung ist die Überprüfung der Genauigkeit der in dem Ionenstrahl-Therapiesystem automatisch durchgeführten Dosisberechnung für eine geplante Bestrahlung in Abhängigkeit von den vorliegenden Basisdaten und den verwendeten Dosisberechnungsalgorithmen, wobei hier zwischen der Bestrahlung eines homogenen und eines inhomogenen Mediums zu unterscheiden ist. In beiden Fällen kann die Überprüfung der Dosisberechnung durch Verwendung eines Phantoms durchgeführt werden, was nachfolgend näher beschrieben wird.

Zur Überprüfung der berechneten Dosis für ein homogenes Medium können im Bestrahlungsplanungsprogramm des Ionenstrahl-Therapiesystems mehrere Meßpunkte, beispielsweise 10 Meßpunkte, in den berechneten Dosisverteilungen bzw. CT-Schnitten definiert werden, an denen die berechnete physikalische Dosis experimentell verifiziert werden soll. Die Verifikation erfolgt in einem Wasserphantom, wobei an den den gewünschten Meßpunkten entsprechenden Koordinaten im Wasserphantom Ionisationskammern positioniert werden. Das Bestrahlungsplanungsprogramm berechnet für die einzelnen Meßpunkte neben den Wasserenergiedosiswerten auch deren Koordinaten in dem verwendeten Phantom. Anschließend wird das Phantom mit den vom Bestrahlungsplanungsprogramm berechneten Steuerparametern bestrahlt, wobei die von dem Ionisationskammern erfaßten Werte in Energiedosiswerte umgerechnet werden, um die berechneten Dosiswerte zu verifizieren.

Die Verifikation wird für mehrere Bestrahlungspläne durchgeführt, wobei bevorzugt sechs typische Bestrahlungspläne verifiziert werden, von denen sich drei Pläne auf konstruierte Zielvolumina im Wasserphantom und drei Pläne auf die Bestrahlung von Patienten beziehen. Die letztgenannten Bestrahlungspläne werden nachfolgend als Standardpatientenpläne verwendet. Die von dem Bestrahlungsberechnungsprogramm berechneten Werte dienen als Bezugswerte für die durchzuführende Konstanzprüfung.

Als Interventionsschwelle wird festgelegt, daß die Abweichung zwischen den berechneten und den gemessenen Bestrahlungsdosiswerten insgesamt, d.h. im Mittel, maximal ±5 % der Dosis des Zielbestrahlungsvolumens betragen darf. Zudem wird festgelegt, daß die maximale Abweichung für einen einzelnen Meßpunkt ±7% betragen darf.

Die oben beschriebene Vorgehensweise bezieht sich insbesondere für die Abnahmeprüfung des Ionenstrahl-Therapiesystems. Für eine Konstanzprüfung genügt die Verifikation von lediglich jeweils zwei der oben beschriebenen Standardpläne, um die Konstanz der berechneten Dosisverteilungen zu überprüfen und diese mit den experimentell zu bestimmenden Dosisverteilungen zu vergleichen. Die Konstanzprüfung sollte vor jedem Bestrahlungsblock durchgeführt werden.

Für die Überprüfung der Genauigkeit der Dosisberechnungen in Abhängigkeit von den Basisdaten, den verwendeten Bestrahlungsberechnungsalgorithmen und der verwendeten Näherung für ein inhomogenes Medium kann ein kugelförmiges Festkörperphantom verwendet werden, welches aus einem wasseräquivalenten Material besteht und aus einzelnen Schichten aufgebaut ist, in die zum Simulieren verschiedener inhomogener Körper unterschiedliche Inhomogenitäten eingesetzt werden können. Diese Inhomogenitäten sind Scheiben, die aus verschiedenen gewebeäquivalenten Materialien (beispielsweise entsprechend dem Material der Lunge, eines weichen oder harten Knochens, von Weichteilen oder von festem Wasser) bzw. lediglich Luft (bei Nichteinsetzen einer Scheibe) bestehen. Auch in diesem Fall werden in dem Phantom bis zu 10 Meßpunkte zur Verifikation definiert, an denen jeweils die Bestrahlungsdosis sowohl von dem Bestrahlungsplanungsprogramm berechnet als auch mit einem Satz von gleichzeitig messenden Ionisationskammern erfaßt und damit verglichen wird.

Es wird vorgeschlagen, zur Abnahmeprüfung drei unterschiedliche Phantomaufbauten zur Untersuchung der berechneten Dosisverteilung hinter Grenzschichten verschiedener Materialien (beispielsweise Luft/Wasser und Knochen/Wasser), in dünnen Inhomogenitäten und in dicken Inhomogenitäten durchzuführen.

Auch bei der Untersuchung der berechneten Dosiswerte für inhomogene Medien wird als Toleranzschwelle vorgeschlagen, eine maximale mittlere Abweichung zwischen den berechneten Dosiswerten und den gemessenen Dosiswerten aller Meßpunkte von ±5 % und eine maximale Abweichung für einen einzelnen Meßpunkt von ±7 % zuzulassen. Zur Konstanzprüfung können die zuvor beschriebenen Tests vor jedem Bestrahlungsblock durchgeführt werden.

Ebenso können die Dosisberechnungen durch Verwendung eines irregulär geformten Prüfphantoms verifiziert werden. In diesem Fall wird ein Prüfphantom benutzt, welches aus wasseräquivalentem Material besteht und beispielsweise einen menschlichen Kopf nachbildet. Wie zuvor beschrieben, werden in dem Phantom bis zu 10 Meßpunkte zur Verifikation definiert. Des weiteren werden Bestrahlungsparameter für ein geeignetes Zielbestrahlungsvolumen in dem Kopfphantom festgelegt und das Prüfphantom mit Hilfe des stereotaktischen Grundrings justiert. Anschließend wird die von dem Bestrahlungsplanungsprogramm des Ionen-Therapiesystems berechneten Werte der Wasserenergiedosis an den gewählten Meßorten anhand der mit den Ionisationskammern an diesen Meßpunkten gemessenen Werten verglichen, wobei wiederum die Abweichung für sämtliche Meßpunkte maximal ±5 % der Dosis des Zielbestrahlungsvolumens betragen darf, während für jeden einzelnen Meßpunkt eine maximale Abweichung von ±7 % zulässig ist. Zur Konstanzprüfung kann dieser Test vor jedem Bestrahlungsblock durchgeführt werden.

Ein weiterer Aspekt bei der Überprüfung der Bestrahlungsplanung betrifft die Prüfung der in dem Ionenstrahl-Therapiesystem eingesetzten bildgebenden Verfahren, um eine korrekte Übertragung der geometrischen Strukturen (z.B. des Zielbestrahlungsvolumens und der Konturen des Patienten) sowie der Planungsparameter von der Bildgebung bis zur Positionierung sicherzustellen. Zu diesem Zweck kann wie bei der Verifikation der berechneten Bestrahlungsdosiswerte im inhomogenen Medium ein Phantom mit scheiben- oder ringförmigen Einsätzen verwendet werden, wobei in diesem Fall die inhomogenen Einsätze zudem unterschiedliche Durchmesser aufweisen können. Von dem Phantom wird ein Bild aufgenommen, und es werden aus den somit erhaltenen CT-Daten für die drei Hauptrichtungen in dem Drehgestell 8 (vgl. Fig. 2) digitale Röntgen-Rekonstruktionen berechnet. Anschließend wird eine Verifikation der Planungsgeometrie mit Hilfe von Röntgenaufnahmen des Röntgenpositioniersystems in den drei Hauptrichtungen durchgeführt. Diese Vorgehensweise kann unter verschiedenen Winkeln der in Fig. 2 gezeigten Patientenliege 9 durchgeführt werden, z.B. unter 0°, 45° und 90°. Auf diese Weise wird die Form und die Lage der Inhomogenitäten in der digitalen Röntgen-Rekonstruktion in bezug auf die Röntgenaufnahmen des Röntgenpositioniersystems verifiziert. Als Toleranzschwellen wird in diesem Fall festgelegt, daß sowohl die maximale positionale Abweichung als auch die maximale Abweichung hinsichtlich der Form der Ringe des Phantoms 2 mm betragen darf. Die Konstanzprüfung kann wiederum vor jedem Bestrahlungsblock durchgeführt werden.

Zur Erhöhung der Betriebssicherheit ist zudem eine Überwachung der Wartung und Weiterentwicklung der in dem Ionenstrahl-Therapiesystem eingesetzten Bestrahlungsplanungsprogramme erforderlich. Nach einer Weiterentwicklung der Bestrahlungsplanungsprogramme kann versehentlich eine falsche Version verwendet werden. Um dies zu vermeiden und sicherzustellen, daß stets die korrekten Versionen der unterschiedlichen Module verwendet werden, wird das Kontrollsystem des Ionenstrahl-Therapiesystems derart ausgestaltet, daß bei jedem Aufruf eines Bestrahlungsplanungsprogramms Versionsnummern mit Datum des jeweiligen Programms angezeigt werden, die von dem Benutzer mit Daten in einem Protokollbuch zu vergleichen sind.

Ebenso muß sichergestellt werden, daß bei einer Weiterentwicklung des Bestrahlungsplanungsprogramms, d.h. bei Vorliegen einer neuen Version, diese nur nach einer erneuten Abnahmeprüfung wirksam wird. Dies kann dadurch geschehen, daß komplette Dosisverteilungen wie zuvor beschrieben für ein homogenes Medium, ein inhomogenes Medium und für ein irregulär geformtes Phantom berechnet und als Sicherungskopie gespeichert werden. Bei Verwendung der neuen Programmversion können diese gespeicherten Dosiswerte als Bezugs- oder Referenzwerte zur Verifikation der Funktionsfähigkeit der neuen Programmversion verwendet werden, da auch bei Verwendung der neuen Programmversion für dasselbe Phantom dieselben Dosiswerte berechnet werden müßten. Diese Überprüfung sollte daher nach jeder Änderung eines Bestrahlungsplanungsprogramms durchgeführt werden.

Ein weiterer Abschnitt des Prüfsystems betrifft die Überprüfung des Rasterscanvorgangs sowie der Dosimetrie.

Dabei betrifft ein erster Prüfaspekt dieses Prüfabschnitts die Teilchenzahlmonitore- bzw. -überwachungsmittel des Ionenstrahl-Therapiesystems, welche bei dem vorliegenden Ausführungsbeispiel - wie bereits beschrieben worden ist - aus großflächigen Ionisationskammern bestehen.

Diesbezüglich muß beispielsweise die Konstanz der Kalibrierfaktoren dieser Ionisationskammern überprüft werden, da sich die Kalibrierfaktoren nur im Rahmen von Luftdichteschwankungen ändern dürfen. Die beiden Ionisationskammern des Rasterscanners sind hinsichtlich der Teilchenzahl pro Überwachungs- oder Monitoreinheit der Ionisationskammern kalibriert. Die Kalibrierung wird durch einen Kalibrierfaktor K beschrieben, der von der Bestrahlungsenergie E der Teilchen und der Schrittweite $\Delta x$ und $\Delta y$ des Rasterscanners abhängt, d.h. $K = K(E, \Delta x, \Delta y)$. Die Kalibrierung der Ionisationskammern erfolgt über eine Dosismessung in einem homogen gerasterten Bestrahlungsfeld, wobei die Abweichungen von den Bezugsbedingungen korrigiert werden und die Anzeige der Ionisationskammer in eine Wasserenergiedosis $D_{scan}$ umgerechnet wird. Der Kalibrierfaktor wird berechnet nach:

$$K(E, \Delta x, \Delta y) = (D_{scan}/M_i) \cdot \Delta x \cdot \Delta y/(S(E)/\rho)$$

mit $(S(E)/\rho)$ = Massenbremsvermögen von $^{12}C$ bei einer Bestrahlungsenergie E, und
M = Monitoreinheiten pro Koordinatenpunkt i der Ionisationskammer.

Der relevante Energiebereich (beispielsweise zwischen 80 MeV/u und 430 MeV/u) wird in mehreren Schritten vermessen. Der Meßort der jeweils überprüften Ionisationskammer befindet sich im Isozentrum 10, wobei die Ionisationskammer bzw. das Dosimeter in einem Festkörperphantom angeordnet ist. Es wird dieselbe Tabelle des Massenbremsvermögens von $^{12}C$ verwendet, die auch der Bestrahlungsplanung zugrunde gelegt wird. Auf diese Weise wird in Abhängigkeit von der Energie E und der Schrittweite $\Delta x, \Delta y$ ein Satz von Kalibrierfaktoren K erhalten, wobei festgelegt wird, daß die Abweichung von den Bezugswerten maximal ±3 % für jeden Kalibrierfaktor betragen darf. Aus dem Satz von Kalibrierfaktoren sollten mindestens drei Werte überprüft werden. Zur Konstanzprüfung sollte dieses Prüfverfahren täglich durchgeführt werden.

Auch die Dosiskonstanz muß überprüft werden, da gleiche vorgewählte Monitoreinheiten der Ionisationskammern stets zu gleichen Dosisanzeigen führen müssen. Es empfiehlt sich daher, die Konstanz der Dosis im Mittelpunkt von würfelförmigen Bestrahlungsvolumina, die von dem Rasterscanner bzw. dessen Magneten 13, 14 erzeugt oder abgetastet werden, in Abhängigkeit von dem Satz der Kalibrierfaktoren der Ionisationskammern zu überprüfen. Zu diesem Zweck wird zum Erhalt von Referenzwerten die Dosis in einem Phantom gemessen, welches derart positioniert ist, daß sich das Isozentrum 10 genau in der Mitte seiner vorderen Oberfläche befindet. Die Bestrahlung erfolgt dabei innerhalb eines Bestrahlungs- oder Dosiswürfels mit 5 cm Kantenlänge, dessen Mittelpunkt als Meßort in 11,3 cm wasseräquivalenter Tiefe angeordnet ist. (Die Berechnung der Steuerdaten zur Erzeugung des Dosiswürfels erfolgt mit Hilfe der CT-basierten Bestrahlungsplanung. Für diesen Schritt ist es zweckmäßiger, das Isozentrum 10 auf den Ort des Strahleintritts in das Wasserphantom zu legen. Des weiteren gestattet die gewählte Meßtiefe eine Vereinheitlichung der Meßausrüstung für die unterschiedlichen Tests.) Die auf diese Weise bestimmte Bestrahlungsdosis wird als Referenzdosis gespeichert. Die nachfolgend gemessenenen tatsächlichen Dosiswerte können dann mit dieser Referenzdosis verglichen werden, wobei eine maximale Abweichung zwischen der tatsächlichen und der nominalen Dosis (Referenzdosis) von ±3 % zulässig ist. Dabei sollte eine tägliche Konstanzprüfung durchgeführt werden.

Auch die Einflußparameter auf die Teilchenzahlmonitore bzw. Ionisationskammern müssen überprüft werden, wobei dabei insbesondere die Abhängigkeit der Kalibrierfaktoren K von der Teilchenfluenz und dem Teilchenfluß überprüft wird. In beiden Fällen sollte eine jährliche Konstanzprüfung durchgeführt werden.

Zur Überprüfung der Abhängigkeit der Kalibrierfaktoren von der Teilchenfluenz wird im wesentlichen dasselbe Verfahren wie bei der Überprüfung der Konstanz der Kalibrierfaktoren durchgeführt. Die Messungen werden in einem Phantom durchgeführt, welches mit einer Fläche von 5 x 5 cm$^2$ bei den Energien 150 MeV/u, 250 MeV/u und 350 MeV/u mit jeweils gleicher Strahlintensität bestrahlt wird. Eine Ionisationskammer wird in der Mitte der bestrahlten Fläche angeordnet, Die Monitorwerte der Ionisationskammer werden so festgelegt, daß sich am Meßort eine Dosis von 0,2 Gy, 0,5 Gy bzw. 1 Gy ergibt. Für diese unterschiedlichen Monitorwerte wird die Übereinstimmung zwischen der tatsächlichen und der nominalen Dosis erfaßt, wobei eine maximale Abweichung von ±3 % zulässig ist. Die Einhaltung dieser engen Toleranz ist sinnvoll und auch durchführbar.

Zur Überprüfung der Abhängigkeit der Kalibrierfaktoren von dem Teilchenfluß wird ebenfalls im wesentlichen dasselbe Verfahren wie bei der Überprüfung der Konstanz der Kalibrierfaktoren angewendet. In diesem Fall wird jedoch die Dosis konstant gehalten und die Strahlintensität jeweils auf einen hohen, mittleren und niedrigen Wert eingestellt, so daß die Übereinstimmung der tatsächlichen Bestrahlungsdosis mit der nominalen Referenzdosis für unterschiedliche Intensitäten überprüft werden kann. Auch hier ist eine maximale Abweichung von ±3 % zulässig.

Hinsichtlich der Ionisationskammern bzw. Teilchenzahlmonitore sollte auch die Abhängigkeit deren Kalibrierfaktoren von der Strahllage überprüft werden. Es wird im wesentlichen dasselbe Verfahren wie bei der Überprüfung der Konstanz der Kalibrierfaktoren durchgeführt, wobei jedoch dieselbe Anordnung wie bei der zuvor beschriebenen Überprüfung der Dosiskonstanz verwendet wird. Die Messungen werden in einem Bestrahlungsvolumen bzw. Bestrahlungswürfel des Rasterscanners 13, 14 mit einer Kantenlänge von 5 cm durchgeführt, jedoch mit einer seitlichen Versetzung von 2 cm und 6 cm. Dabei werden die Monitorwerte der Ionisationskammern derart festgelegt, daß sich in der Mitte des Bestrahlungsvolumens eine Bestrahlungsdosis von 1 Gy ergibt. Bei der Überprüfung der Anzeigen der Ionisationskammern sollte sich der seitlich gemessene Wert nicht mehr als 3 % von dem in der Mitte gemessenen Wert unterscheiden. Auch in diesem Fall wird eine jährliche Konstanzprüfung empfohlen.

Ein weiterer Prüfaspekt dieses Prüfabschnitts betrifft die Überprüfung der Dosisverteilung des Rasterscanners 13, 14, wobei sowohl die Tiefendosisverteilung als auch die Dosisquerverteilung überprüft wird.

Die Homogenität der Tiefendosisverteilung wird in Abhängigkeit von einer ausgewählten Bestrahlungsenergie und ausgewählten Monitorwerten pro Bestrahlungsenergiewert der verwendeten Ionisationskammern überprüft, da die Tiefendosishomogenität entscheidend von der gewählten Energie und deren Konstanz abhängt. Zu diesem Zweck werden mit den Rasterscannermagneten 13, 14 in einem Phantom erneut quader- oder würfelförmige Bestrahlungsvolumina erzeugt, wobei für jeden Koordinatenpunkt einer Schicht (Energie) eine konstante Teilchenbelegung, jedoch pro Schicht eine unterschiedliche Teilchenbelegung derart verwendet wird, daß sich in dem Bestrahlungswürfel eine homogene Dosisverteilung ergibt. Mehrere Dosimeter (Ionisationskammern), beispielsweise 10 Ionisationskammern, messen in unterschiedlichen wasseräquivalenten Tiefen, wobei die Ionisationskammern derart positioniert werden, daß nicht mehrere Ionisationskammern hintereinander bestrahlt werden. Die Kantenlängen der Bestrahlungswürfel betragen beispielsweise 2,5 cm, 5 cm und 10 cm, wobei die Messungen der Ionisationskammern für Tiefen der Mittelpunkte der jeweiligen würfelförmigen Bestrahlungsvolumens von 5 cm, 12,5 cm bzw. 20 cm durchgeführt werden. Die Monitorwerte werden aus der Bestrahlungsplanung in der Weise festgelegt, daß sich in der Mitte des jeweiligen Bestrahlungsvolumens eine durch die Bestrahlungsplanung vorgegebene Bestrahlungsdosis ergibt. Durch Vergleich der tatsächlichen Meßwerte mit den Referenzwerte kann die Schwankungsbreite der Anzeigen der Ionisationskammern überprüft werden. Eine maximale Abweichung von ±5 % ist tolerierbar. Bei Überschreiten dieser Toleranzgrenze muß in das System eingegriffen werden, um die zu große Abweichung zu korrigieren. Zur Konstanzprüfung sollte das oben beschriebene Prüfverfahren vor jedem Bestrahlungsblock durchgeführt werden.

Die Dosisquerverteilung des Rasterscanners wird in Abhängigkeit von der Energie überprüft, um sicherzustellen, daß die Homogenität des Rasterscanverfahrens bei allen verwendeten Bestrahlungsenergien gewährleistet ist. In diesem Fall wird bei festen Ionisationskammer-Monitorwerten und jeweils unterschiedlichen Bestrahlungsenergien (z.B. 100 MeV/u, 150 MeV/u, 200 MeV/u, 250 MeV/u, 300 MeV/u und 350 MeV/u) und Strahlfeldern die Bestrahlungsdosis senkrecht zur Strahlrichtung mit mehreren gleichzeitig messenden Ionisationskammern festgestellt. Gleichzeitig wird vor den Dosimetern bzw. Ionisationskammern frei Luft eine Schwärzungsverteilung auf einem Verifikationsfilm erzeugt. Mit dem Rasterscanner 13, 14 werden Flächen mit einer Seitenfläche von beispielsweise 5 cm, 10 cm und 18 cm erzeugt, wobei die Bestrahlungsdosis jeweils ca. 1 Gy betragen soll. Es wird die Standardabweichung der korrigierten Anzeigen der Ionisationskammern bzw. der Verifikationsfilmschwärzung innerhalb des Bestrahlungsfeldes überprüft, wobei eine maximale Abweichung von den Bezugswerten von ±5 % tolerierbar ist. Nicht tolerierbare Abweichungen von den Bezugswerten werden korrigiert, um eine Anpassung an die tatsächlich vorliegenden Meßbedingungen zu erzielen. Eine Konstanzprüfung sollte vor jedem Bestrahlungsblock durchgeführt werden, wobei in diesem Fall die Verwendung des Verifikationsfilms mit Überwachung der Schwärzung dieses Verifikationsfilms genügt.

Ein weiterer Prüfaspekt dieses Prüfabschnitts betrifft die Überprüfung der Feldgeometrie beim Rasterscanverfahren, wobei die Abhängigkeit der räumlichen Lage eines bestimmten Bestrahlungsvolumens des Rasterscanners 13, 14 von gewählten Bestrahlungsenergien überprüft wird. Zu diesem Zweck werden von dem Rasterscanner 13, 14 würfel- oder quaderförmige Bestrahlungsvolumina erzeugt, wobei eine konstante Teilchenbelegung für jeden Koordinatenpunkt einer Schicht (Energie), jedoch eine unterschiedliche Belegung pro Schicht derart verwendet wird, daß sich eine homogene Dosisverteilung in dem Bestrahlungswürfel ergibt. Unter diesen Bedingungen wird ein keilförmiges Festkörperphantom bestrahlt, hinter dem sich ein verifikationsfilm befindet. Anschließend wird die Position der Verifikationsfilmschwärzung relativ zu dem Mittelpunkt der Bestrahlung festgestellt.

Bei der Messung betragen die Kantenlängen der Bestrahlungsfelder beispielsweise 4 cm, 7 cm und 12 cm, während die Ausdehnung der Bestrahlungsquader oder -würfel in Strahlrichtung 2,5 cm, 5 cm und 10 cm betragen. Die Messungen werden dabei für wasseräquivalente Tiefen des Mittelpunkts der jeweiligen Bestrahlungsvolumina von 5 cm, 12,5 cm bzw. 20 cm durchgeführt. Die Monitorwerte der Dosimeter bzw. Ionisationskammern werden derart aus der Bestrahlungsplanung festgelegt, daß sich in der Mitte des Bestrahlungsvolumens eine durch die Bestrahlungsplanung vorgegeben Bestrahlungsdosis ergibt. Als Feldgrenzen werden die Orte definiert, an denen der Randabfall der Schwärzung bei 50 % des Plateauwerts liegt. Die Lage der distalen Feldgrenzen sowie der in Strahlrichtung gesehen seitlichen Feldgrenzen werden überprüft und mit Bezugswerten verglichen. Eine Abweichung von 2mm in jeder Richtung ist tolerierbar, ansonsten muß eine Korrektur des Systems erfolgen, um das System an die tatsächlich vorliegenden Meßbedingungen anzupassen. Dieses Prüfverfahren sollte zur Konstanzprüfung vor jedem Bestrahlungsblock durchgeführt werden, wobei hier eine Auswahl von jeweils drei Bedingungen aus den Kombinationen der zuvor beschriebenen Bedingungen genügt.

Schließlich betrifft ein weiterer Prüfaspekt dieses Prüfabschnitts die Verifikation des Gesamtsystems, um die Genauigkeit der applizierten Bestrahlungsdosis hinsichtlich ihrer Höhe und Räumlichkeit bei jedem zu bestrahlenden Patienten verifizieren zu können, so daß eine korrekte Zusammenarbeit der einzelnen Komponenten des Systems gewährleistet wird. Dabei wird zwischen der Bestrahlung eines homogenen Mediums und eines inhomogenen Mediums unterschieden.

Im ersten Fall wird wie bei der zuvor beschriebenen Verifikation der Übereinstimmung von berechneten und gemessenen Dosisverteilungen für ein homogenes Medium ein homogenes Phantom verwendet und im wesentlichen dasselbe Verfahren durchgeführt, jedoch mit der Ausnahme, daß nunmehr individuelle Patientenbestrahlungspläne als Grundlage dienen. Für sämtliche Meßpunkte wird der Unterschied zwischen der berechneten Bestrahlungsdosis und der gemessenen Bestrahlungsdosis ermittelt, wobei wiederum eine mittlere Abweichung für sämtliche Meßpunkte von 5 % und eine Abweichung für einen einzelnen Meßpunkt von 7 % tolerierbar ist. Zur Konstanzprüfung sollte dieser Test vor jedem Bestrahlungsblock durchgeführt werden.

Zur Überprüfung der Genauigkeit bei Bestrahlung eines zu bestrahlenden inhomogenen Mediums wird wiederum ein inhomogenes Phantom verwendet, wobei in diesem Fall als einmalige Vorbereitung für ein halbkugelförmiges Phantom aus einem festen, wasseräquivalentem Material mit einem Radius von beispielsweise 8 cm eine Bestrahlungsplanung durchgeführt wird. Für die Bestrahlungsplanung befindet sich der Mittelpunkt des Phantoms im Isozentrum 10 und die Halbkugel des Phantoms ist der Einstrahlrichtung entgegengewandt. In das Phantom können unterschiedliche Inhomogenitäten, beispielsweise in Form von Scheiben mit einem Durchmesser von jeweils 3 cm, eingelegt werden, wobei bevorzugt sieben verschiedene Materialien oder Inhomogenitäten mit folgenden Dichten verwendet werden:

| Nr. | Dichte | |
|---|---|---|
| 1 | 0,001 | (Luft) |
| 2 | 0,30 | (Lunge) |
| 3 | 1,035 | (festes Wasser) |
| 4 | 0,92 | (Fett) |
| 5 | 1,05 | (Muskel) |
| 6 | 1,14 | (weicher Knochen) |
| 7 | 1,84 | (harter Knochen) |

Das geplante Zielbestrahlungsvolumen ist für drei unterschiedliche Einstrahlrichtungen mit einem Einstrahlwinkel von 0°, +45° und -45° jeweils eine 2cm dikke Schicht innerhalb des Halbkugelphantoms, die unmittelbar an die flache Seite der Halbkugel angrenzt, so daß die distale Lage des Bestrahlungsvolumens mit der hinteren Flachseite übereinstimmt. Die im Zielbestrahlungsvolumen geplante homogene Bestrahlungsdosis beträgt 1 Gy. Mit diesen Steuerdaten für die Steuerung des Rasterscanners werden die Bestrahlungen mit den drei Einstrahlrichtungen durchgeführt, wobei sowohl im Zielbestrahlungsvolumen als auch hinter jeder Inhomogenität ein Dosimeter (d.h. eine Ionisationskammer) positioniert ist, deren Anzeige überwacht wird. Die ermittelte Energiedosis in allen Meßpunkten sollte innerhalb des Zielbestrahlungsvolumens nicht die Schwelle 1 Gy $\pm$5 % überschreiten, während 5 cm hinter dem Zielbestrahlungsvolumen eine maximale Abweichung von $\pm$10 % von der berechneten und auf das Zielbestrahlungsvolumen bezogenen Bestrahlungsdosis tolerierbar ist. Zudem ist für sämtliche Meßpunkte erneut eine mittlere Abweichung der gemessenen Bestrahlungsdosis von $\pm$5 % und für jeden einzelnen Meßpunkt eine maximale Abweichung von $\pm$7 % tolerierbar. Zur Konstanzprüfung sollte dieses Prüfverfahren vor jedem Bestrahlungsblock durchgeführt werden.

**Patentansprüche**

1. Verfahren zur Überprüfung einer Notabschaltung eines Schwerionenstrahl-Therapiesystems, wobei das Schwerionenstrahl-Therapiesystem umfaßt

- mindestens eine Ionenquelle (1),
- eine Beschleunigereinrichtung (2, 5) zum Beschleunigen der Ionen der Ionenquelle (1) in Form eines Behandlungsstrahls (11),
- ein Strahlführungssystem (6, 8), um den Behandlungsstrahl (11) von der Beschleunigereinrichtung (2, 5) mindestens einem Bestrahlungsplatz zur Behandlung eines Patienten zuzuführen, wobei das Strahlführungssystem (6, 8) mindestens eine Strahlführungsleitung (6) umfaßt, und

- eine in dem Strahlführungssystem (6, 8) angeordnete Rasterscannereinrichtung mit vertikalen Ablenkmitteln (13) und horizontalen Ablenkmitteln (14) zur vertikalen bzw. horizontalen Ablenkung des Behandlungsstrahl (11) senkrecht zu seiner Strahlrichtung, so daß der Behandlungsstrahl (11) von der Rasterscannereinrichtung auf ein Isozentrum (10) des Bestrahlungsplatzes abgelenkt wird und eine bestimmte, das Isozentrum (10) umgebende Fläche abtastet, und

wobei eine Überprüfung einer Notabschaltung durchgeführt wird,
**dadurch gekennzeichnet,**
**daß** eine manuelle und eine automatische Notabschaltung unter automatischer Überwachung aller sicherheitsrelevanter Geräteparameter und unter Anzeigen aller sicherheitsrelevanter Zustände an Konsolen eines technischen Kontrollraumes der Therapie und eines Hauptkontrollraumes des Gesamtsystems einer Schwerionenstrahl-Therapieanlage durch eine Interlockeinheit überprüft wird, wobei die Interlockeinheit im Betrieb mindestens folgendes überwacht:

- Signale der Endschalter der beweglichen Teile in der Strahlführung,
- Zustände von Magnetnetzgeräten der Rasterscannmagnete,
- Spannungsversorgungen von Ionisationskammern,
- Datenüberläufe der Datenübertragung,
- Intensitätsgrenzwerte und Gleichlauf der Ionisationskammern,
- Elektronik von Strahlpositionsmeßeinrichtungen und der Strahlposition selbst,
- Hochspannungen und Gasflüsse von Detektoren,
- Funktionsbereitschaft von Rechnerprogrammen,
- Konsolen des technischen Kontrollraums der Therapie und des Hauptkontrollraums,

und wobei mehrere Meßpunkte eines Phantoms überprüft werden, wobei auf eine ausreichende Genauigkeit der Bestrahlungsdosisdaten geschlossen wird, falls die mittlere Abweichung zwischen berechneten und gemessenen Werten der Bestrahlungsdosis für sämtliche Meßpunkte einen ersten Toleranzwert von $\pm$ 5% und für jeden einzelnen Meßpunkt die Abweichung zwischen der für diesen Meßpunkt berechneten und gemessenen Bestrahlungsdosis einen zweiten Toleranzwert von $\pm$ 7%

nicht überschreitet.

2. Verfahren nach Anspruch 1, **dadurch gekennzeichnet, daß** Signale der Endschalter der Außenanlagen aller beweglichen Teile der Strahlführung vorzugsweise Sollpositionen von Vakuumteilen, Vakuumschiebern und Strahldiagnoseelementen, von einem Umlenkmagneten bis zum Behandlungsraum überwacht werden und zur Überprüfung im Testfall simuliert werden.

3. Verfahren nach einem der Ansprüche 1 oder 2, **dadurch gekennzeichnet, daß** der Zustand der Impulszentralansteuerung überwacht wird und zur Prüfung im Testfall simuliert wird.

4. Verfahren nach einem der Ansprüche 1 bis 3, **dadurch gekennzeichnet, daß** die Funktion der Impulszentrale überwacht wird und zur Überprüfung im Testfall simuliert wird.

5. Verfahren nach einem der Ansprüche 1 bis 4, **dadurch gekennzeichnet, daß** der Zustand des Magnetnetzgerätes des horizontalen Scannermagneten überwacht wird und zur Überprüfung im Testfall simuliert wird.

6. Verfahren nach einem der Ansprüche 1 bis 5, **dadurch gekennzeichnet, daß** der Zustand des Magnetnetzgerätes des vertikalen Scannermagneten überwacht wird und zur Überprüfung im Testfall simuliert wird.

7. Verfahren nach einem der Ansprüche 1 bis 6, **dadurch gekennzeichnet, daß** die Soll-/Istwerte und eine Fortschaltung des Bestrahlungspunktes der Scannermagnete überwacht werden und zur Überprüfung im Testfall simuliert werden.

8. Verfahren nach einem der Ansprüche 1 bis 7, **dadurch gekennzeichnet, daß** von der Intensitätsmeßelektronik der Ionisationskammern ein Summensignal über die Spannungsversorgung, die Signale elektronischer Wandler von analogen Meßgrößen in digitale Werte, die Signale eines Überlaufs der Datenübertragung und die Signale einer Intensitätsüberlastung überwacht werden und zur Überprüfung im Testfall simuliert werden.

9. Verfahren nach einem der Ansprüche 1 bis 8, **dadurch gekennzeichnet, daß** eine Freigabe der Bestrahlung durch eine medizinische Bedienkonsole überwacht wird und zur Überprüfung im Testfall simuliert wird.

10. Verfahren nach einem der Ansprüche 1 bis 9, **dadurch gekennzeichnet, daß** jede Quelle eines Interlock- oder Notabschaltfalles einzeln simuliert wird und eine Auslösung der Notabschaltung, sowie Signale zur Notabschaltung des Schwerionenstrahls von einer Interlockeinheit erzeugt werden.

11. Verfahren nach Anspruch 10, **dadurch gekennzeichnet, daß** die Interlockeinheit und ihre Funktion täglich überprüft wird und eine fehlende Auslösung eines Notabschaltfalles bei einem vorliegenden Interlockstatus eines Gerätes keine Freigabe einer Patientenbehandlung erfolgt.

12. Verfahren nach einem der Ansprüche 1 bis 11, **dadurch gekennzeichnet, daß** eine Notabschaltung durch menschliche Intervention jederzeit gewährleistet wird.

13. Verfahren nach einem der Ansprüche 1 bis 12, **dadurch gekennzeichnet, daß** eine Überprüfung einer Notabschaltung durch den Patienten mittels eines Handschalters und unter Öffnen einer Patientenfixierung durchgeführt wird.

14. Verfahren nach einem der Ansprüche 1 bis 13, **dadurch gekennzeichnet, daß** eine Überprüfung eines manuellen Notabschalters an der medizinischen Bedienkonsole durchgeführt wird.

15. Verfahren nach einem der Ansprüche 1 bis 14, **dadurch gekennzeichnet, daß** eine Überprüfung einer manuellen Notabschaltung täglich durchgeführt wird und bei fehlerhafter manueller Notabschaltung keine Freigabe einer Patientenbehandlung erfolgt.

16. Verfahren nach einem der Ansprüche 10 bis 15, **dadurch gekennzeichnet, daß** in Verbindung mit dem Überprüfen einer Interlockeinheit die Anzeigen aller Alarmbedingungen überprüft werden.

17. Verfahren nach einem der Ansprüche 1 bis 16, **dadurch gekennzeichnet, daß** vor jedem Bestrahlungsblock und nach jeder Änderung eines Kontrollsystems oder eines Programms eine Überprüfung der Anzeigen aller sicherheitsrelevanter Zustände an den Konsolen eines technischen Kontrollraumes der Therapie und eines Hauptkontrollraums durchgeführt wird.

18. Verfahren nach einem der Ansprüche 1 bis 17, **dadurch gekennzeichnet, daß** nach einem Ausbleiben einer Anzeige sicherheitsrelevanter Zustände keine Freigabe für eine Patientenbehandlung erfolgt.

**Claims**

1. A method of checking an emergency shutdown of a heavy ion beam therapy system,

the heavy ion beam therapy system comprising

- at least one ion source (1),
- an accelerator device (2, 5) for accelerating the ions from the ion source (1) in the form of a treatment beam (11),
- a beam guidance system (6, 8), to guide the treatment beam (11) from the accelerator device (2, 5) to at least one irradiation station for treating a patient, the beam guidance system (6, 8) comprising at least one beam guidance channel (6), and
- a raster scanning device arranged in the beam guidance system (6, 8) and having vertical deflection means (13) and horizontal deflection means (14) for the vertical and horizontal deflection of the treatment beam (11) perpendicular to its beam direction, so that the treatment beam (11) is deflected by the raster scanning device onto an isocenter (10) of the irradiation station and scans a specific area surrounding the isocenter (10), and

a check of emergency shutdown being carried out, **characterized in that**
an interlock unit checks manual and automatic emergency shutdown with automatic monitoring of all the safety-relevant device parameters and with displays of all the safety-relevant states on consoles of a technical therapy control room and a main control room for the entire system of a heavy ion beam therapy installation, the interlock unit monitoring at least the following during operation:

- signals from the limit switches on the moving parts in the beam guidance system,
- states of magnetic power supplies of the raster scanner magnets,
- power supplies of ionization chambers,
- data overflows of the data transmission,
- intensity limiting values and synchronism of the ionization chambers,
- electronics of beam position measuring devices and the beam position itself,
- high voltages and gas flows of detectors,
- functional readiness of computer programs,
- consoles in the technical therapy control room and the main control room,

and a plurality of measurement points of a phantom being checked, adequate accuracy of the irradiation dose data being assumed if the average deviation between calculated and measured values of the irradiation dose for all the measurement points does not exceed a first tolerance value of $\pm 5\%$ and, for each individual measurement point, the deviation between the calculated and measured irradiation dose for this measurement point does not exceed a second tolerance value of $\pm 7\%$.

2. The method as claimed in claim 1, **characterized in that** signals from the limit switches of the external installations of all the moving parts of the beam guidance, preferably desired positions of vacuum parts, vacuum slides and beam diagnostic elements, from a deflection magnet to the treatment room, are monitored and are simulated for checking in the test case.

3. The method as claimed in either of claims 1 and 2, **characterized in that** the state of the pulse central control system is monitored and is simulated for testing in the test case.

4. The method as claimed in one of claims 1 to 3, **characterized in that** the function of the pulse central system is monitored and is simulated for checking in the test case.

5. The method as claimed in one of claims 1 to 4, **characterized in that** the state of the magnetic power supply of the horizontal scanner magnet is monitored and is simulated for checking in the test case.

6. The method as claimed in one of claims 1 to 5, **characterized in that** the state of the magnetic power supply of the vertical scanner magnet is monitored and is simulated for checking in the test case.

7. The method as claimed in one of claims 1 to 6, **characterized in that** the desired/actual values and stepping of the irradiation point of the scanner magnets are monitored and are simulated for checking in the test case.

8. The method as claimed in one of claims 1 to 7, **characterized in that** a total signal relating to the power supply, the signals relating to electronic converters from analog measured variables into digital values, the signals relating to an overflow in the data transmission and the signals relating to an intensity overload are monitored by the intensity measuring electronics of the ionization chambers and are simulated for checking in the test case.

9. The method as claimed in one of claims 1 to 8, **characterized in that** the release of the irradiation is monitored by a medical operating console and is simulated for checking in the test case.

10. The method as claimed in one of claims 1 to 9, **characterized in that** each source of an interlock or emergency shutdown case is simulated individually, and the triggering of the emergency shutdown, and also signals for the emergency shutdown of the heavy ion beam are generated by an interlock unit.

**11.** The method as claimed in claim 10, **characterized in that** the interlock unit and its functions are checked daily, and upon lack of triggering an emergency shutdown case in the event of an interlock status being present in a device, the system is not released for patient treatment.

**12.** The method as claimed in one of claims 1 to 11, **characterized in that** an emergency shutdown by human intervention is ensured at any time.

**13.** The method as claimed in one of claims 1 to 12, **characterized in that** a check is carried out on an emergency shutdown by the patient by means of a hand switch and with a patient fixing being opened.

**14.** The method as claimed in one of claims 1 to 13, **characterized in that** a check is carried out on a manual emergency shutdown on the medical operating console.

**15.** The method as claimed in one of claims 1 to 14, **characterized in that** a check of a manual emergency shutdown is carried out daily and, in the event of faulty manual emergency shutdown, the system is not released for patient treatment.

**16.** The method as claimed in one of claims 10 to 15, **characterized in that**, in conjunction with the checking of an interlock unit, the displays of all the alarm conditions are checked.

**17.** The method as claimed in one of claims 1 to 16, **characterized in that** before each irradiation block and after each change in a monitoring system or a program, a check is carried out on the displays of all the safety-relevant states on the consoles of a technical control room for the therapy and of a main control room.

**18.** The method as claimed in one of claims 1 to 17, **characterized in that** in the event of a display of safety-relevant states remaining absent, the system is not released for patient treatment.

## Revendications

**1.** Procédé de surveillance de l'arrêt d'urgence d'un système de thérapie par faisceaux d'ions lourds, le système de thérapie par faisceaux d'ions lourds comprenant

- au moins une source d'ions (1),
- un dispositif d'accélération (2, 5) pour accélérer les ions de la source d'ions (1) sous la forme d'un faisceau de traitement (11),
- un système de guidage de faisceau (6, 8) pour amener le faisceau de traitement (11) du dispositif d'accélération (2, 5) à au moins un poste d'irradiation pour le traitement d'un patient, le système de guidage de faisceau (6, 8) comprenant au moins un conduit de guidage de faisceau (6), et

- un système de scanner à balayage disposé dans le système de guidage de faisceau (6, 8), avec des moyens de déviation verticale (13) et des moyens de déviation horizontale (14) pour dévier verticalement, voire horizontalement, le faisceau de traitement (11) perpendiculairement à sa direction de faisceau, d'une manière telle que le faisceau de traitement (11) soit dévié par le système de scanner à balayage sur un isocentre (10) du poste d'irradiation et balaie une surface déterminée autour de l'isocentre (10),

surveillance de l'arrêt d'urgence étant effectuée **caractérisé par le fait qu'**on contrôle à l'aide d'une unité d'interlock un arrêt d'urgence manuel et un arrêt d'urgence automatique, en surveillant automatiquement tous les paramètres de sécurité de l'appareil et en affichant tous les états importants sur le plan de la sécurité sur des consoles d'une salle de contrôle technique du traitement et d'une salle de contrôle principale de l'ensemble d'un système de thérapie par faisceaux d'ions lourds, sont, l'unité d'interlock, en service, surveillant au moins :

- les signaux des contacteurs de fins de course des éléments mobiles sur le trajet du faisceau,
- les états des unités d'alimentation aimantées des aimants du scanner à balayage aimanté,
- les alimentations en tension des chambres à ionisation,
- les débordements de données dans la transmission de données,
- les valeurs limites d'intensité et la synchronisation des chambres à ionisation
- l'électronique de dispositifs de mesure de position du faisceau et la position du faisceau elle-même
- les hautes tensions et les flux gazeux de détecteurs,
- la disponibilité fonctionnelle de programmes de calcul
- les consoles de la salle de contrôle technique du traitement et de la salle de contrôle principale,

plusieurs points de mesure d'un fantôme étant contrôlés,
la précision des données de dose de rayonnement étant considérée suffisante lorsque l'écart moyen entre les valeurs calculées et les valeurs mesurées pour la dose de traitement, pour tous les points de mesure, ne dépasse pas une première valeur de tolérance de ±5% et que, pour chaque point de mesure individuel, l'écart entre les doses de rayonnement calculées et mesurées pour ce point ne dépasse pas une seconde valeur de tolérance de ±7%.

2. Procédé selon la revendication 1, **caractérisé par le fait qu'**on surveille les signaux des contacteurs de fin de course des matériels extérieurs de tous les éléments mobiles du système de guidage de faisceau, de préférence les positions de consigne d'éléments à vide, de distributeurs à vide et d'éléments de diagnostic de faisceau, depuis un aimant de déviation jusqu'à la salle de traitement et on simule ceux-ci pour le contrôle au cours de tests.

3. Procédé selon la revendication 1 ou 2, **caractérisé par le fait qu'**on surveille l'état du générateur central d'impulsions et on simule celui-ci pour le contrôle au cours de tests.

4. Procédé selon la revendication 1 à 3, **caractérisé par le fait qu'**on surveille le fonctionnement du générateur central d'impulsions et on simule celui-ci pour le contrôle au cours de tests.

5. Procédé selon la revendication 1 à 4, **caractérisé par le fait qu'**on surveille l'état de l'unité d'alimentation aimantée de l'aimant de scanner horizontal aimanté et on simule celui-ci pour le contrôle au cours de tests.

6. Procédé selon la revendication 1 à 5, **caractérisé par le fait qu'**on surveille l'état de l'unité d'alimentation aimantée de l'aimant de scanner vertical aimanté et on simule celui-ci pour le contrôle au cours de tests.

7. Procédé selon la revendication 1 à 6, **caractérisé par le fait qu'**on surveille les valeurs de consigne/valeurs réelles et un adressage du point d'irradiation des aimants de scanner vertical et on simule ceux-ci pour le contrôle au cours de tests.

8. Procédé selon la revendication 1 à 7, **caractérisé par le fait qu'**on surveille un signal global de l'électronique de mesure d'intensité des chambres à ionisation sur l'alimentation en tension, les signaux de convertisseurs électroniques de signaux grandeurs de mesure analogiques en valeurs numériques, les signaux de débordement de la transmission de données et les signaux d'une surcharge en intensité et on simule ceux-ci pour le contrôle au cours de tests.

9. Procédé selon la revendication 1 à 8, **caractérisé par le fait qu'**on surveille un déclenchement de l'irradiation à partir d'une console d'utilisateur médicale et on simule celui-ci pour le contrôle au cours de tests.

10. Procédé selon la revendication 1 à 9, **caractérisé par le fait qu'**on simule séparément chaque source d'un cas d'arrêt par interlock ou d'arrêt d'urgence et un déclenchement de l'arrêt d'urgence et que des signaux pour l'arrêt d'urgence du faisceau d'ions lourds sont produits par une unité d'interlock.

11. Procédé selon la revendication 10, **caractérisé par le fait qu'**on procède quotidiennement à un contrôle de l'unité d'interlock et de son fonctionnement et en cas d'absence d'arrêt d'urgence dans un état d'interlock d'un appareil le traitement d'un patient est bloqué.

12. Procédé selon une des revendications 1 à 11, **caractérisé par le fait qu'**un arrêt d'urgence est toujours assuré par une intervention humaine.

13. Procédé selon une des revendications 1 à 12, **caractérisé par le fait qu'**on surveille un arrêt d'urgence par le patient à l'aide d'un commutateur manuel et en ouvrant une sangle d'attache du patient.

14. Procédé selon une des revendications 1 à 13, **caractérisé par le fait qu'**on contrôle un commutateur manuel d'arrêt d'urgence sur la console d'utilisateur médicale.

15. Procédé selon une des revendications 1 à 14, **caractérisé par le fait qu'**on procède quotidiennement à un contrôle d'arrêt d'urgence manuel et en cas de défaut d'arrêt d'urgence manuel, le traitement du patient est bloqué.

16. Procédé selon une des revendications 10 à 15, **caractérisé par le fait qu'**en liaison avec le contrôle de l'unité d'interlock on contrôle les informations de toutes les conditions d'alarme.

17. Procédé selon une des revendications 1 à 16, **caractérisé par le fait qu'**avant chaque bloc d'irradiation et après chaque modification d'un système de contrôle ou d'un programme, on procède à un contrôle des affichages de tous les états importants sur le plan de la sécurité sur les consoles d'une salle de contrôle technique du traitement et d'une salle de contrôle principale.

18. Procédé selon une des revendications 1 à 17, **caractérisé par le fait qu'**en cas d'absence d'un affichage d'état important sur le plan de la sécurité, le traitement du patient est bloqué.

# Fig. 1

Fig. 2

EP 1 152 796 B1